# EUROPEAN PATENT SPECIFICATION

(11) **EP 1 520 859 B1**
(45) Date of publication and mention of the grant of the patent: **14.04.2010**
(21) Application number: 04030374.5
(22) Date of filing: 12.01.1995
(51) Int. Cl.: C07K 14/00, A61K 38/00, C12N 9/04

(54) **Hydrophilic signal oligopeptides and methods of therapeutic use**
Hydrophile Signal-Oligopeptide und Verfahren der therapeutischen Anwendung
Oligopeptides signaux hydrophiles et leurs procédés d'utilisation à des fins thérapeutiques

(30) Priority: 14.01.1994 US 182248
(43) Date of publication of application: 06.04.2005
(62) Divisional of application: 95908522.6
(73) Proprietor: Rath, Matthias, Dr. med., 6422 PC Heerlen (NL)
(72) Inventor: Rath, Matthias, Dr. med., 6422 PC Heerlen (NL)
(74) Representative: Kleiner, Christoph

(56) References cited:
- US-A- 4 554 101
- CHIN, DANIEL J. ET AL: "Nucleotide sequence of 3-hydroxy-3-methyl-glutaryl coenzyme A reductase, a glycoprotein of endoplasmic reticulum" NATURE (LONDON, UNITED KINGDOM) , 308, 613-17 CODEN: NATUAS; ISSN: 0028-0836, 1984, XP008094019
- LUSKEY, KENNETH L. ET AL: "Human 3-hydroxy-3-methylglutaryl coenzyme A reductase. Conserved domains responsible for catalytic activity and sterol-regulated degradation" JOURNAL OF BIOLOGICAL CHEMISTRY , 260(18), 10271-7 CODEN: JBCHA3; ISSN: 0021-9258, 1985, XP002981007
- SKALNIK, DAVID G. ET AL: "The nucleotide sequence of Syrian hamster HMG-CoA reductase cDNA" DNA , 4(6), 439-44 CODEN: DNAADR; ISSN: 0198-0238, 1985, XP008093880
- 19000101, 1 January 1900 (1900-01-01), XP008093979

## Description

### Field of Invention

The present patent claims further specific signal oligopeptides and their therapeutic use.

### References

1. Watson JD, Crick FHC. 1953. Nature 171:737
2. Rath. M. 1993. J. Orthorn. Medicine 8:11
3. Rath, M. 1994. J Appl. Nutr. (in Press)

### Background

The discovery of the genetic code four decades ago defined the principles by which genes encode for proteins (1). The protein code, however, the biological language by which protein act and interact has remained obscure. Now the protein code is discovered (2,3). The interaction of hormones and other ligands with their respective receptors, of enzymes with protein substrates, of adhesive proteins with integrins, and of antibodies with antigens as well as other protein actions and interactions are determined by the same structure/function principles and the same biological language. For a very limited number of proteins the hydrophobic interior of the protein forms the active center which also determines their function. In the vast majority of proteins, however, biological activity is primarily mediated via specific surface structures which mediate their specific functions. The structure/function principles determining these protein actions are discovered (1) and they are hereafter referred to as the protein code

### Summary of the invention

Uke the human language the protein code consists of letters words and sentences. The letters (amino acids) and sentences (complete three dimensional protein) had been known before. The new discovery are the protein words or verbs. These protein verbs are represented by signal oligopeptides which are localized on the surface of the protein and are represented by the hydrophyticity maxima of the protein. These signal oligopeptides are enriched in charged amino acids in a versatile arrangement with neutral spacer ammo acids The specific signal character of these oligopeptides is determined by a characteristic combination of conformation and charge within the same signal sequence

Uke in the human language the whole sentence (complete three dimensional protein) is needed to determine the specific and complete action of any given protein. In the human language eliminating or changing the verb of a sentence renders the whole sentence meaningless. Similarly, blocking the protein code verbs (signal oligopeptides) can be therapeutically used to block the undesired action or interaction of an entire protein.

The discovery of the protein code provides the rationale for deciphering the communication code of diseases. Infectious diseases, cancer, cardiovascular and other diseases develop by means of one or more pathogenicity-mediating proteins. Blocking the signal oligopeptides of these proteins allows the specific therapeutic interception of a pathological communication and thereby blocks disease propagation.

From Luskey and Stevens, 1985, The journal of Biological Chemistry 260: 10271-10277, it is known the full length cDNA for human 3-hydroxy-3-methylglutaryl coenzyme A reductase, the membrane bound glycoprotein that regulates cholesterol synthesis.

Synthetic analogs to signal oligopeptides can be used therapeutically in several ways. First, synthetic analogues to signal oligopeptides can be used as competitive inhibitors of pathological communication. Second, synthetic analogs to signal oligopeptides can be used as vaccines. This second therapeutic approach makes use of the fact that signal oligopeptides on the surface of the protein are identical with the antigenicity determining epitopes of this protein. Thus, antibodies are interceptors of metabolic communication. By binding to the signal oligopeptide of a protein antibodies and other mediators of immune response reduce or block its metabolic interaction. If synthetic analogs to signal oligopeptides are used as vaccines it is necessary to render these peptides antigenic and to allow their discrimination as 'non-self'. Synthetic analogs to signal oligopeptides can be rendered immunogenic by coupling them to haptens and by other conventional methods.

The invention is based on methods by which oligopeptide sequences can be rendered immunogenic. This method is based on the discovery of the primary structural principles determining immunogenicity. The discrimination between self and non-self between species and between individuals is primarily based on amino acid residue substitutions or other residue variations within the signal oligopeptide sequences of a protein. By making use of this discovery effective therapeutic signal oligopeptide can be rapidly produced in the following way signal oligopeptides of a given protein in one species are the antigenicity determinants of this protein in another species To block the action of a pathogenicity-mediating protein in the treatment of a human disease the synthetic signal oligopeptide vaccines are designed by copying corresponding amino acid signal sequences from another species. A glucagon signal oligopeptide vaccine for the treatment of diabetic patients would be based on glucagon signal sequences from rabbits, sheep, mice or other species. A titration of the therapeutic efficiency is possible: The greater the genetic and evolutionary distance of the selected species to humans the greater its antigenicity and consequently the greater its therapeutic efficiency as a vaccine.

### Detailed Description of the Figures

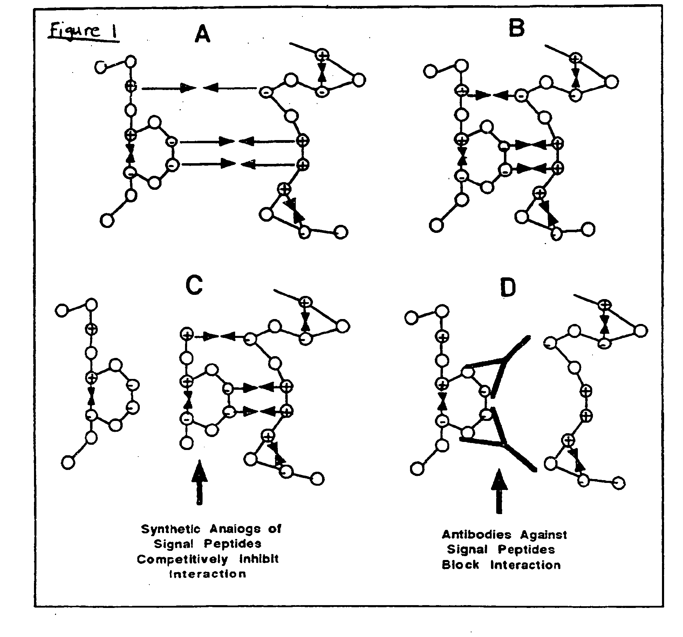

Figure 1. This figure illustrates several of the protein code principles. *Legibility:* Within the amino acid sequence of a protein one or more oligopeptides represent the words - or verbs - of the protein code which mediates its action and interaction. *Accessibility*: The signal sequences of a protein are enriched in charged amino acids and represent the hydrophylicity maxima within the potential sequence (Figure 1A). *Variability*: An infinite number of possible combinations between amino acids with different charges as well as neutral resides provide the variability for differentiated metabolic communication. *Specificity:* The specificity of a signal sequence is the result of a characteristic combination of charge distribution and structural conformation within the signal oligopeptide sequence. Oppositely charged amino acids can attract each other thereby enhancing confirmational specificity. Signal oligopeptides mediate specific information transfer to their metabolic counterparts (Figure 1 B). *Differentiation*: Substitution, deletion or other amino acid residue variations within the signal sequence of a protein enable differentiation between 'self' and 'non-self'. Signal sequences are the antigenic epitopes of a protein and are responsible for potential immune responses when exposed to other organisms. *Interception:* Direct and indirect Peptide Interception Therapy (PIT) can be used to intercept undesired or pathological communication and thereby block the disease. In direct PIT synthetic analogs to signal oligopeptides can be used to competitively inhibit the interaction of proteins (Figure 1C). Indirect PIT makes use of synthetic oligopeptides rendered immunogenic. These vaccines stimulating the production of antibodies which block pathological communication pathways not only in the prevention and treatment of infectious diseases but also in the therapy of neoplastic diseases, metabolic disorders and other diseases (Figure 1D).

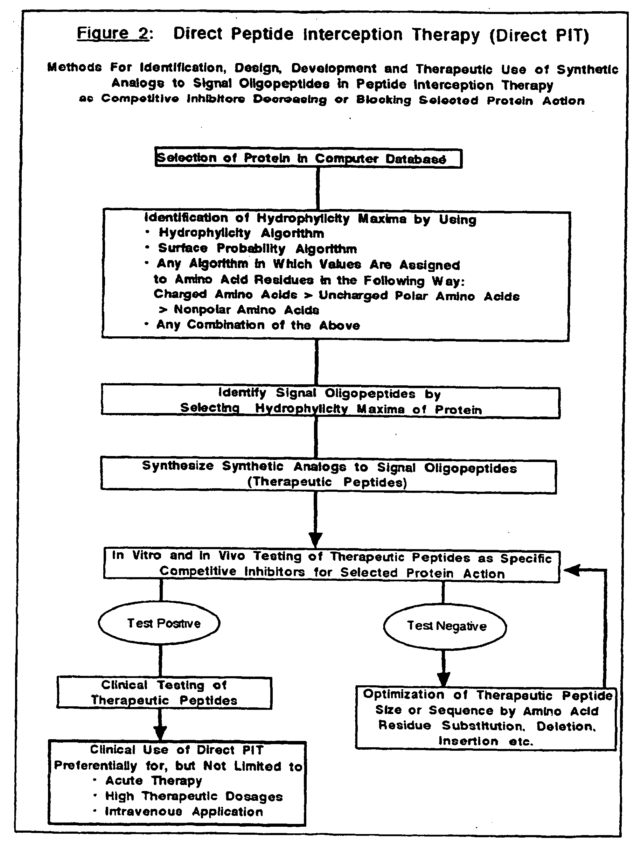

Figure 2. Fundamentals of Peptide interception Therapy (PIT). Proteins are essential carriers of specific metabolic information: moreover, proteins are frequently mobile which makes them ideal and versatile communication molecules. This figure illustrates several key elements of PIT: PIT is an interception therapy. To *promote protein action*, the entire three dimensional protein structure (protein sentence) is required To *intercept protein action,* only the signal oligopeptide (verb) has to be blocked by direct or indirect PIT.
Figure 2 shows the principles of Direct Peptide interception Therapy (Direct PIT) including the methods for identification, design, development and therapeutic use of synthetic analogs to signal oligopeptides in Peptide Interception Therapy as direct competitive inhibitors of selected protein actions.

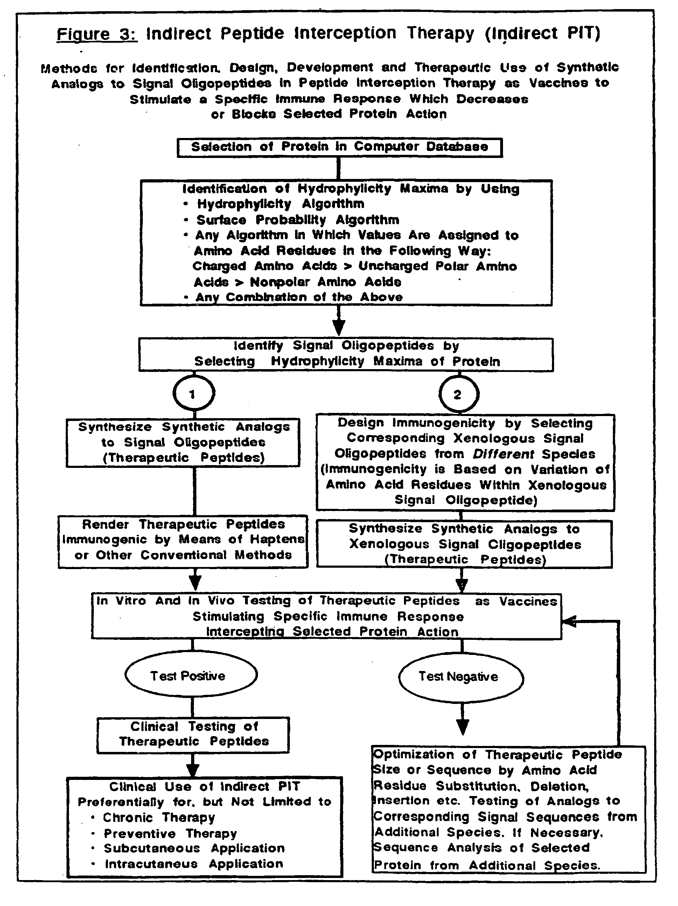

Figure 3. This figure shows the principles of Indirect Peptide Interception Therapy (Indirect PIT) including the methods for identification, design, development and therapeutic use of synthetic analogs to signal oligopeptides in Peptide Interception Therapy as vaccines to stimulate a specific immune response with the aim to decrease or block selected protein actions.

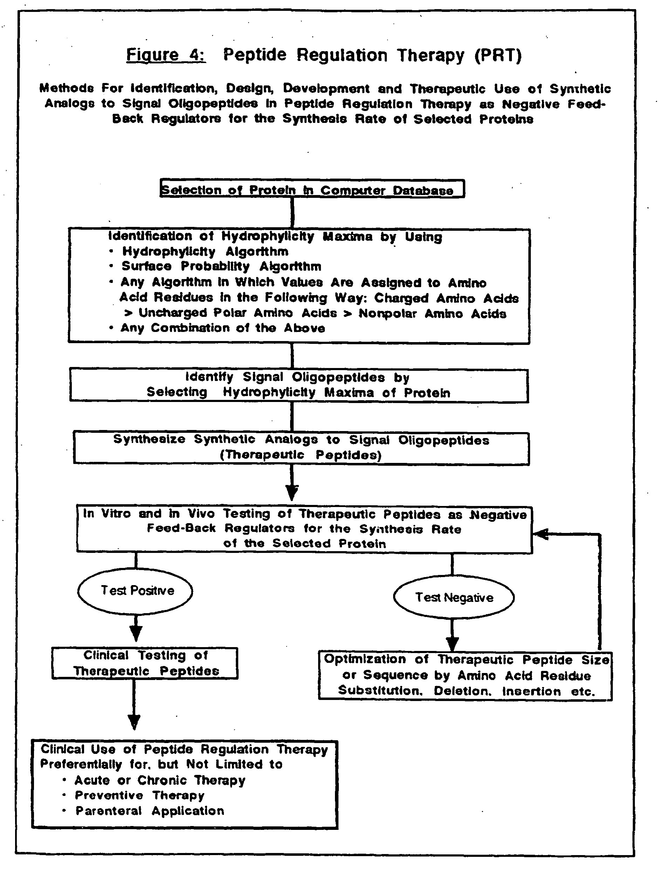

Figure 4. This figure shows the principles of Peptide Regulation Therapy (PRT) including the methods for identification, design, development and therapeutic use of synthetic analogs to signal oligopeptides in Peptide Regulation Therapy as negative feed-back regulators for the synthesis rate of selected proteins.

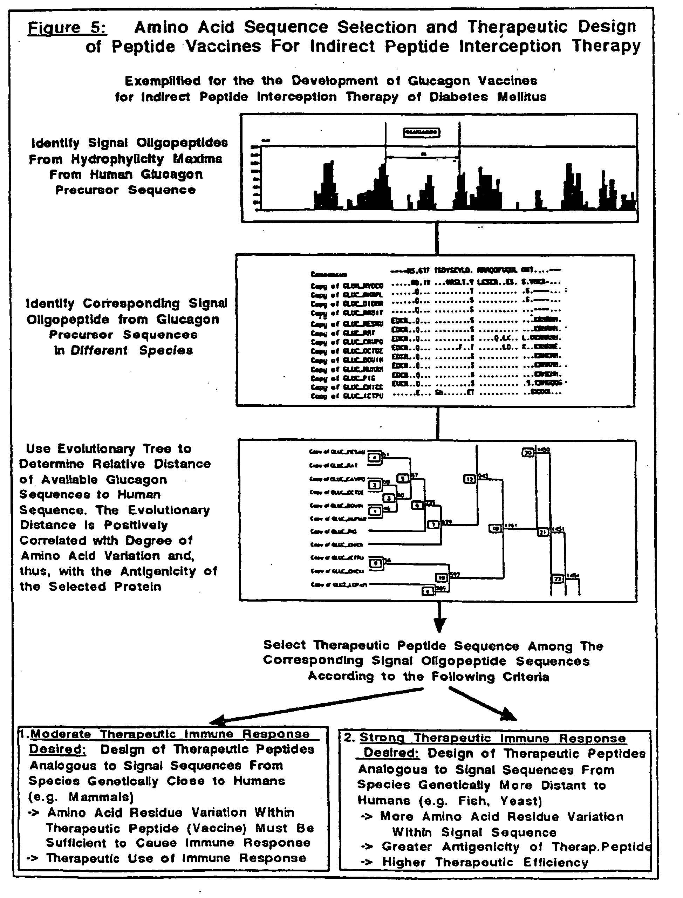

Figure 5. Ammo acid sequence selection and therapeutic design of peptide vaccines for Indirect Peptide interception Therapy, exemplified for the development of glucagon vaccines in clinical therapy of diabetes mellitus.

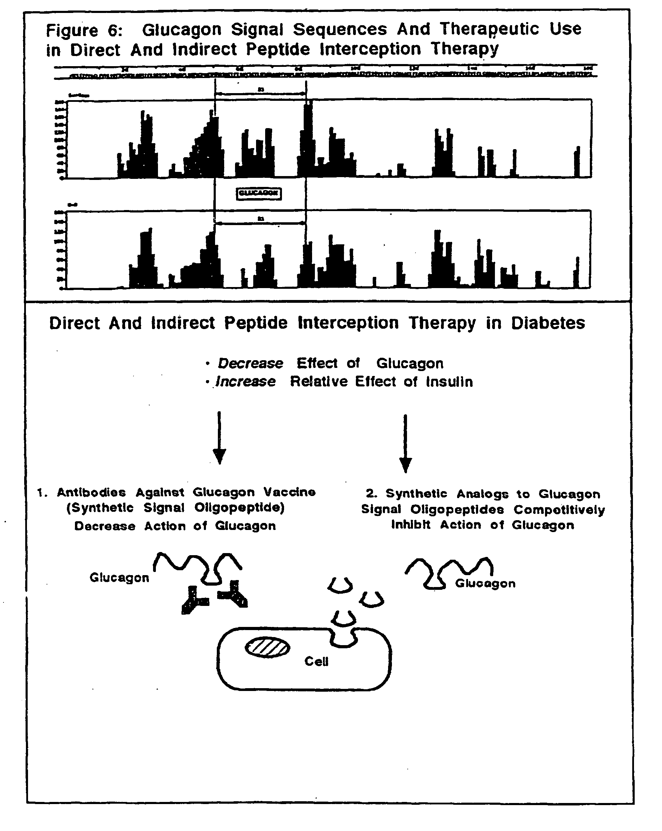

Figure 6. Peptide therapy in diabetes. This figure shows the potential signal sequences of proglucagon and therapeutic alternatives for peptide therapy in diabetic patients. Conventional treatment in diabetes focuses on an increased availability of insulin. Peptide therapy enables an alternative approach. Synthetic analogs to the signal sequences of glucagon can be therapeutically used to attenuate the effect of this insulin antagonist. Note the three hydrophilicity peaks in the glucagon precursor sequence (A,B,C). The mature glucagon hormone is activated by contact and charge redistribution within the peaks A and C. PIT therapy could have two distinct targets: Blocking charge redistribution of precursor molecule (PIT for Peak A or C) prevents activation of precursor to mature hormone. PIT targeting peak B prevents the action of the glucagon hormone. Vaccination with synthetic glucagon signal oligopeptides rendered immunogenic could become a baseline treatment for diabetic patients.

### 1. Determining Principles of the Protein Code

The protein code is determined by the following principles:
*1. Universality.* The protein code is universally valid. Proteins are important carriers of metabolic information in living organisms. The protein code is the biological language for protein-mediated information transfer during health and disease. The protein code is the underlying communication principle for the interaction of antigens with antibodies, enzymes with substrates, receptors with ligands, adhesion molecules with integrins and other forms of protein communication
*2. Compatibility.* The protein code is compatible with other elements of biological communication. The protein code and the genetic code are closely connected. The genetic code determines the structure of proteins and, thereby, their function. The protein code determines the relation between structure and function within a protein sequence and, thereby, the specific biological action. Specific and efficient action of a protein are responsible for the advantage of a gene and, therefore, for the survival of the genetic information encoding for the protein.
*3. Legibility.* The protein code is composed of letters, sentences and words. The protein code *letters* are represented by the amino acid residues of a protein. The protein code sentence is represented by the complete three dimensional structure of the protein which ultimately determines its action. The decisive signal of protein communication is mediated by the protein code words. These protein code words are represented by one or more signal oligopeptides within a protein sequence. In the human language verbs are the action words within a sentence. Signal oligopeptides are the verbs of the protein code and determine the action of the protein. Interference with these signal oligopeptides can lead to substantial modification or loss of the biological message of a protein.
*4. Accessibility.* The protein code has to be accessible. Protein information transfer and protein interaction is dependent on the accessibility of the signal sequence. Signal oligopeptides are generally represented by the regions of maximum hydrophilicity on the surface of the protein molecule. Critical determinants for these hydrophilicity maxima is an enrichment of the cationic amino acid residues arginine and lysine and/or the anionic amino acid residues glutamate and aspartate.
*5. Variability-* The protein code requires a sufficient reservoir of structural variations encoding for a large number of diverse signals. The protein code is based on optimum variability. The protein code is based on an almost infinite number of possible signal oligopeptide structures. A specific arrangement of charged amino acid residues in combination with a variable number of uncharged residues within signal tripeptides, tetrapeptides, pentapeptides, hexapetides or larger polypeptides provide the necessary number of possible residue combinations for a giant signal reservoir.

A new type of signals is represented by oligopeptides which obtain their characteristic conformation by a specific arrangement of oppositely charged amino acid residues within this oligopeptide sequence. These residues with opposite charge can attract each other thereby modulating a characteristic folding of this signal sequence. In the signal sequence RGD the cationic residue arginine and the anionic residue aspartate attract each other leading to a characteristic fording of this tripeptide around the 'spacer' residue glycine.
*6. Specificity.* The protein code is specific. The specific and reliable metabolic function of a protein is dependent on the specificity of its biological signal sequences The signal character of a specific oligopeptide is determined by a characteristic combination of electrical charge with structural conformation. Within a protein, RGD and analogous tripeptides can serve as strong primary anchors while the specific biological message is mediated by additional longer and more complex signal oligopeptides.
*7. Differentiation.* The protein code provides the basis for the immunological differentiation between individuals and species. Substitutions, omissions, and other variations of one or more amino acid residues within the signal sequence of the protein enable an organism to differentiate between 'selt' and 'non-self'. Thus, the protein code comprises the basic language of immunology.
*B. Diversification.* The protein code is a key for individual development within a species as well as for the evolutionary diversification of species. The effectiveness of signal oligopeptides to mediate specific biological messages were the ultimate criterion for the evolutionary advantage of a protein and, thus, for the evolutionary survival of the gene encoding for it. Genetic mutations leading to the substitution of one or more amino acid residues within a signal oligopeptide sequence were an economic and therefore frequent mechanism to modulate and differentiate protein action and thereby promoting revolutionary diversification.
*9. Interception.* The protein code is interceptable. The signal oligopeptides of a protein are identical with the potential antigenic determinants. Antibodies and other mediators of immune response are interceptors of specific biological communication. Signal oligopeptides as promoters of differentiated protein communication and immune response mediators as interceptors can form a sophisticated network of biological communication. Decoding the physiologic aspects of this communication network will lead to a precise understanding of millions of metabolic interactions including the principles for development and differentiation of the body. Decoding the pathophysiological aspects of this communication network will lead to the therapeutic control of many diseases and eventually to their eradication as causes of human mortality.
*10. Regulation.* The protein code could also provide a missing link in the regulation of protein synthesis. The signal sequences of a protein may have important feed-back functions directly or indirectly modulating the synthesis rate of this protein.

### II. Principles of Peptide Interception Therapy (PIT) and of Peptide Regulation Therapy (PRT)

1. The metabolic interaction of proteins is primarily modulated by one or more oligopeptide signal sequences which determine the metabolic interaction of this protein. Peptide Interception Therapy (PIT) is defined as the specific therapeutic interception of pathological or undesired protein actions and interactions by the therapeutic use of synthetic analogs to-the signal oligopeptide sequences of this protein. Peptide Regulation Therapy (PRT) makes therapeutic use of synthetic analogs to signal oligopeptide sequences to decrease the synthesis rate of an undesired protein via feed-back mechanisms.
2. Peptide interception Therapy uses the discovery of the protein code: To *promote* protein action, the entire three dimensional protein structure (protein sentence) is required. To *intercept* protein action, only the signal oligopeptide (protein verb) has to be therapeutically blocked.
3. These signal oligopeptide sequences are located on the surface of the protein and are represented by the hydrophilicity maxima within the amino acid sequence of the protein. The signal oligopeptide sequences of a protein can be identified from its primary structure by use of a protein data base in combination with a suitable algorithm, such as for hydrophilicity or surface probability. In this algorithm the highest hydrophilicity or probability values have to be assigned to the charged amino acids lysine, arginine, aspartate and glutamate followed by asparagine and glutamine.
4. Synthetic analogs to signal oligopeptide sequences can be therapeutically used. An unlimited number of signal oligopeptide analogs can be synthesized covering the entire sequence of a selected hydrophylicity peak or parts of it.
5. Signal tetrapeptides, pentapeptides, hexapeptides and longer peptides represent primary candidates for specific peptide therapy. Shorter peptides, such as the tripeptide RGD, are less specific and ubiquitous side effects limit their broad therapeutic use.
6. Peptide Interception Therapy (PIT) can be used in two principal ways: Direct PIT uses synthetic analogs to signal oligopeptides as competitive inhibitors of pathological or undesired metabolic interaction. Indirect PIT uses these synthetic analogs as vaccines to stimulate the production of specific antibodies. In indirect PIT the antibodies, not the therapeutic peptide itself, function as interceptors of protein communication.
7. Direct Peptide Interception Therapy (Direct PIT) uses synthetic analogs to Signal oligopeptides as direct competitive inhibitors for undesired protein communication. Direct blocking of pathogenicity mediating protein communication leads to the control of the related disease or clinical condition This therapeutic approach would be preferentially used in acute conditions, e.g. antithrombotic or fibrinolytic therapy. Direct PIT would be preferentially used intravenously in higher therapeutic dosages of the synthetic peptide. Poly-oligopeptide analogs, time release delivery systems and other modifications of the peptide delivery mechanism can be used to extend the range of possible therapeutic applications.
8. Indirect Peptide Interception Therapy (Indirect PIT). Today vaccines are essentially limited to prophylaxis and therapy of infectious diseases. Indirect PIT enables the extension of the preventive and therapeutic use of vaccines to all areas of medicine. The great advantage of oligopeptide vaccines, compared to conventional vaccines, is that not the entire protein is used as a vaccine but only synthetic analogs to one or more of the signal oligopeptides of the selected protein.
9. Indirect PIT is based on the therapeutic use of antibodies produced by the patient's own immune system against the signal sequences of proteins mediating pathogenicity for undesired metabolic action. Indirect PIT is preferentially used for preventive therapy, for the treatment of chronic conditions or as adjuncts to direct PIT or other forms of acute therapy.
10. Indirect PIT makes use of two newly discovered principles of immunology: First, the signal oligopeptides of a given protein are identical with its potential antigenicity determining regions (antigenic epitopes). Second, the primary mechanism determining antigenicity between different individuals and different species are amino acid residue substitutions, omissions and other residue variations within the signal oligopeptide sequence(s) of a protein.
11. Therapeutic peptide design for indirect PIT is based on the following principle: Signal oligopeptides of a given protein in one species are the antigenic epitopes of this protein for the immune system of another species. To block the action of a pathogenicity-mediating protein in the treatment of a human disease, the amino acid residue sequence of the oligopeptide vaccines should be homologous to the signal sequences of the same protein - but from another species. A glucagon signal oligopeptide vaccine for the treatment of diabetic patients would be based on the glucagon signal sequences from rabbits, sheep, mice or other species. The aim of this residue manipulation is to create an antigenic epitope without compromising the ability of the antibodies produced to effectively block the metabolic interaction of the protein. This therapeutic approach mimics nature's way to discriminate between 'self' and non-self and make therapeutic use of it.
12. The designer of therapeutic compounds for indirect PIT can also make use of the evolutionary clock. The further apart two species are in evolution, the more amino acid residue mutations occurred, including mutations in the signal oligopeptide sequences, and the more antigenic is the therapeutic peptide composed of this sequence. A titration of the therapeutic efficiency of indirect PIT is possible: Indirect PIT therapy with Synthetic analogs to glucagon signal oligopeptides from a *fish* species are more effective than those from a *mammalian* species In blocking *human* glucagon action.
13. Antigenicity required for effective indirect PIT therapy can also be stimulated in conventional ways, e.g. by coupling the therapeutic peptides to defined haptens or other immunogenic compounds enhancing 'non-self' recognition
14. A third therapeutic mechanism using synthetic analogs to signal oligopeptides is as feed-back regulators for protein synthesis. This form of therapy is designated Peptide Regulation Therapy (PRT). The synthesis rate of many proteins is determined by the amount of protein end-product available. The signal sequences of pathogenicity mediating proteins can be used to decrease the synthesis of this protein via direct or indirect feedback mechanisms.

### III. Advantages of Peptide Therapy

***1**. **Efficacy***. Peptide therapy is a highly effective form of treatment. The discovery of the peptide code provides the rationale for deciphering the communication code of human diseases and allows the interception of pathological interactions with maximum effectiveness.
***2.*** ***Specificity.*** Peptide therapy enables maximum therapeutic specificity. Based on the precise understanding of the structure/function relation of specific protein signals, peptide therapy allows therapeutic targeting with unprecedented specificity.
***3.*** ***Safety.*** Peptide therapy is extremely safe. The use of synthetic analogs to physiologic compounds essentially eliminates the problem of toxicity. Possible undesired biological side-effects should be controllable by optimizing the length and composition of the therapeutic peptide.
***4.*** ***Savings in therapeutic research and development.*** Time and expenses for the development of therapeutic peptides is a fraction of conventional therapeutic research and development. Identification of potential therapeutic peptides takes minutes; in vitro screening of potential peptides is a matter of weeks: animal studies should provide first in vivo results within a few months. Most importantly, the unprecedented specificity and safety background of peptide therapy will allow clinical studies without delay.
***5.*** ***Advantage of peptide therapy compared to conventional therapies**.* The advantages of peptide therapy become even more obvious when this novel therapeutic approach is compared to conventional therapies or alternative future therapies*. Conventional drug therapy* is frequently compromised by an unknown therapeutic mechanism and by a wide range of side effects and considerable toxicity. *Gene therapy* is compromised by limited availability, its technological requirements and its high costs, which restricts its therapeutic application to exclusive areas in the foreseeable future. *Heterologous or synthetic antibody therapy,* the therapeutic application of antibodies produced outside of the patient's body, can cause incalculable adverse reactions by the patient's, immune system against these 'foreign' antibodies. In contrast, peptide therapy makes elegant use of the patient's own immune system thereby excluding adverse immunological reactions.

### IV. Therapeutic Applications of Peptide Therapy

***Metabolic disorders**.* A novel therapeutic area for peptide therapy is the treatment of metabolic disorders. The potential of peptide therapy is exemplified here for the treatment of diabetes and hypertension.

*Diabetes.* Conventional diabetic therapy aims at an increased availability of insulin, Peptide therapy allows a novel and alternative approach by inhibiting the action of glucagon, the insulin antagonist. The inhibition of glucagon can be accomplished by using the therapeutic peptides analogous to the glucagon signal sequence for direct competitive inhibition or as a vaccine (Figure 5).

The following oligopeptide signal sequences of Hydroxyl-methyl-glutaryl coenzyme A reductase as well as their use for therapeutic puposes are claimed in this patent:

### HYOROXY-METHYL-GLUTARYL COENZYME A REDUCTASE

### INFORMATION FOR SEQUENCE ID NO 42

(A) LENGTH
(B) TYPE amino acid
(D) TOPOLOGY linear

### INFORMATION FOR SEQUENCE ID NO 44

(A) LENGTH:
(B) TYPE: amino acid
(D) TOPOLOGY linear
-E-L-S-R-E-S-R-E-G-R-

| Table 1: The Maxima of the Following Algorithms -or Modifications Thereof-Can Be Used to Determine Potential Signal Oligopeptides in the Primary Structure of a Protein From a Protein Sequence Database | | |
|---|---|---|
| Amino Acid Residue | A. Hydrophylicity Algorithm* | B. Surface Probability Algorithm** |
| Arginine | 3.0 | 9.5 |
| Aspartate | 3.0 | 8.1 |
| Glutamate | 3.0 | 8.4 |
| Lysine | 3.0 | 9.7 |
| Aspartate or Asparagine | 1.6 | 8.0 |
| Glutamate or Glutamine | 1.6 | 8.4 |
| Serine | 0.3 | 6.5 |
| Asparagine | 0.2 | 7.8 |
| Glutamine | 0.2 | 8.4 |
| Glycine | 0.0 | 4.8 |
| Proline | 0.0 | 7.5 |
| Threonine | -0.4 | 7.0 |
| Alanine | -0.5 | 4.9 |
| Histidine | -0.5 | 6.6 |
| Cysteine | -1.0 | 2.6 |
| Methionine | -1.3 | 4.8 |
| Valine | -1.5 | 3.6 |
| Isoleucine | -1.8 | 3.4 |
| Leucine | -1.8 | 4.0 |
| Tyrosine | -2.3 | 7.6 |
| Phenylalanine | -2.5 | 4.2 |
| Tryptophan | -3.4 | 5.1 |
| | | |
| C. Algorithm Based on the Following Amino Acid Categories: | | |
| | | |
| 1. Highest Values Assigned to Charged Amino Acids: Aspartate, Glutamate, Lysine, Arginine, Histidine | | |
| 2. Medium Values Assigned to Uncharged Polar Amino Acids: Asparagine, Glutamine, Glycine, Cysteine, Serine, Threonine, Tyrosine | | |
| 3. Lowest Values Assigned to Non Polar Amino Acids: Alanine, Valine, Leucine, Isoleucine, Proline, Phenylalanine, Methionine, Tryptophan | | |
| | | |
| * According to Hopp TP, Woods. KR. 1981. Proc. Natl. Acad. Sci. USA. 78: 3824- 3828. | | |
| ** Boger. Proceedings of the 1988 Miami Bio/Technology Winter Symposium. 10 Oxford and Washington. IRL Press. | | |

| Table 2 | |
|---|---|
| Methods of Use disclosed For the Signal Sequences in This Patent And For Those in Preceding Patents With Serial No. 07/997,734; 07/997,727; and 08/045,394 | |
| A. Synthetic Analogs to Human Signal Sequences Can Be Used | B. Synthetic Analogs to Protein Signal Sequences From *Other Species* Can Be Used |
| 1. As Therapeutic Agents for the Direct Competitive Inhibition of Selected Protein Interaction (Direct PIT) | As Therapeutic Agents (Vaccines)in the Prevention and Treatment of Human Diseases. Stimulating a Specific Immune Response Which Blocks or Decreases the Action of the Selected Protein in the Human Body. |
| 2. As Therapeutic Agents in Feed-Back Regulation With the Therapeutic Aim to Decrease the Synthesis Rate of the Selected Protein (PRT). | 2. As Therapeutic Agents According to 1 and 2 in Column A in the Treatment of Diseases in the Respective Animal Species. |
| 3. As Therapeutic Agents in Combination With Haptens or other Conventional Immunogens or Adjuvants as Vaccines Stimulating a Specific Immune Response Which Blocks or Decreases the Action of the Selected Protein (Indirect PIT) | 3. As Antigens to Produce Antibodies Against the Selected Protein for *In Vitro* Diagnostic Purposes in the Respective Animal Species. |
| 4. As Antigens to Produce Antibodies Against the Selected Human Protein for *In Vitro* Diagnostic Purposes. | |

| Table 3 | |
|---|---|
| | |
| The Therapeutic Use of Synthetic Signal Sequences as Vaccines (Indirect Peptide Interception Therapy) | |
| | |
| A. Diseases Mediated by *Xenologous* Proteins (Infectious Diseases) | B. Diseases Mediated by *Human* Proteins (e. g. Metabolic Disorders. Chronic Human Diseases) |
| | |
| 1. Choose Signal Sequence From Xenologous Protein (e.g. Toxin) as Basis For Vaccine in Humans | 1. Choose from Different Species the Signal Sequences Corresponding to the Selected Human Proteins (Xenologous Signal Sequence) as Basis For Vaccine |
| | |
| 2. Use Vaccines the Prevention and Treatment of Infectious Diseases | 2. Use Vaccines in the Prevention and Treatment of Metabolic Disorders, Cardiovascular Diseases and other Human Diseases |

### SEQUENCE LISTING

<110> Rath, Matthias
<120> Hydrophilic signal oligopeptides and methods of therapeutic use
<130> 1305-04
<140> EP 04030374.5
   <141> 2004-12-21
<160> 360
<170> PatentIn version 3.2
<210> 1
   <211> 9
   <212> PRT
   <213> Artificial
<220>
   <223> Farnesyl synthetase
<400> 1
   Asp Val Tyr Ala Gln Glu Lys Gln Asp
   1 5
<210> 2
   <211> 9
   <212> PRT
   <213> Artificial
<220>
   <223> Farnesyl synthetase
<400> 2
   Asp Val His Asn Gln Glu Lys Gln Asn
   1 5
<210> 3
   <211> 6
   <212> PRT
   <213> Artificial
<220>
   <223> Farnesyl synthetase
<400> 3
   Glu Ile Arg Arg Glu Arg
   1 5
<210> 4
   <211> 11
   <212> PRT
   <213> Artificial
<220>
   <223> Farnesyl synthetase
<400> 4
   Glu Asp Glu Met Gly His Phe Glu Ile Gly Asp
   1 5 10
<210> 5
   <211> 11
   <212> PRT
   <213> Artificial
<220>
   <223> Farnesyl synthetase
<400> 5
   Glu Asp Glu Leu Gly His Pro Glu Lys Gly Asp
   1               5                   10
<210> 6
   <211> 11
   <212> PRT
   <213> Artificial
<220>
   <223> Farnesyl synthetase
<400> 6
   Ala Ser Leu Leu Ala Tyr Gly Met Pro Lys Glu
   1               5                   10
<210> 7
   <211> 8
   <212> PRT
   <213> Artificial
<220>
   <223> Farnesyl synthetase
<400> 7
   Glu Pro Arg Lys Gln Asp Ala Asp
   1               5
<210> 8
   <211> 8
   <212> PRT
   <213> Artificial
<220>
   <223> Farnesyl synthetase
<400> 8
   Glu Pro Arg Lys Gln Asp Ala Glu
   1               5
<210> 9
   <211> 11
   <212> PRT
   <213> Artificial
<220>
   <223> Farnesyl synthetase
<400> 9
   Asn Lys Thr Val Glu Gln Leu Gly Gln Glu Glu
   1               5                   10
<210> 10
   <211> 11
   <212> PRT
   <213> Artificial
<220>
   <223> Farnesyl synthetase
<400> 10
   Asp Asp Ile Met Asp Ser Ser Leu Thr Arg Arg
   1               5                   10
<210> 11
   <211> 11
   <212> PRT
   <213> Artificial
<220>
   <223> Farnesyl synthetase
<400> 11
   Asp Asp Ile Met Asp Ser Ser Tyr Thr Arg Arg
   1               5                   10
<210> 12
   <211> 11
   <212> PRT
   <213> Artificial
<220>
   <223> Farnesyl synthetase
<400> 12
   Asp Asp Met Met Asp Lys Ser Ile Thr Arg Arg
   1               5                   10
<210> 13
   <211> 10
   <212> PRT
   <213> Artificial
<220>
   <223> Farnesyl synthetase
<400> 13
   Lys Leu Tyr Cys Arg Glu Gln Pro Tyr Tyr
   1               5                   10
<210> 14
   <211> 10
   <212> PRT
   <213> Artificial
<220>
   <223> Farnesyl synthetase
<400> 14
   Lys Ser His Phe Arg Asn Glu Lys Tyr Tyr
   1               5                   10
<210> 15
   <211> 8
   <212> PRT
   <213> Artificial
<220>
   <223> Farnesyl synthetase
<400> 15
   Arg Phe Thr Glu Lys Arg Tyr Lys
   1               5
<210> 16
   <211> 8
   <212> PRT
   <213> Artificial
<220>
   <223> Farnesyl synthetase
<400> 16
   Arg Tyr Thr Glu Lys Arg Tyr Lys
   1               5
<210> 17
   <211> 8
   <212> PRT
   <213> Artificial
<220>
   <223> Farnesyl synthetase
<400> 17
   Lys Phe Ser Leu Lys Lys His Ser
   1               5
<210> 18
   <211> 11
   <212> PRT
   <213> Artificial
<220>
   <223> Farnesyl synthetase
<400> 18
   Lys Val Asp Leu Ser Lys Phe Ser Leu Lys Lys
   1               5                   10
<210> 19
   <211> 11
   <212> PRT
   <213> Artificial
<220>
   <223> Farnesyl synthetase
<400> 19
   Lys Lys His Ser Phe Ile Val Thr Phe Lys Thr
   1               5                   10
<210> 20
   <211> 11
   <212> PRT
   <213> Artificial
<220>
   <223> Farnesyl synthetase
<400> 20
   Asp Gly Glu Lys Glu His Ala Asn Ala Lys Lys
   1               5                   10
<210> 21
   <211> 11
   <212> PRT
   <213> Artificial
<220>
   <223> Farnesyl synthetase
<400> 21
   Asp Gly Glu Lys Glu His Ala Asn Ala Leu Lys
   1 5 10
<210> 22
   <211> 11
   <212> PRT
   <213> Artificial
<220>
   <223> Farnesyl synthetase
<400> 22
   Thr Asp Glu Lys Asp Leu Lys Gln Ala Arg Asp
   1 5 10
<210> 23
   <211> 10
   <212> PRT
   <213> Artificial
<220>
   <223> Farnesyl synthetase
<400> 23
   Lys Ile Gly Thr Asp Ile Gln Asp Asn Lys
   1 5 10
<210> 24
   <211> 10
   <212> PRT
   <213> Artificial
<220>
   <223> Farnesyl synthetase
<400> 24
   Lys Val Gly Thr Asp Ile Gln Asp Asn Lys
   1 5 10
<210> 25
   <211> 8
   <212> PRT
   <213> Artificial
<220>
   <223> Farnesyl synthetase
<400> 25
   Gln Arg Ala Thr Pro Glu Gln Tyr
   1               5
<210> 26
   <211> 8
   <212> PRT
   <213> Artificial
<220>
   <223> Farnesyl synthetase
<400> 26
   Leu Arg Ala Thr Pro Gln Gln Arg
   1               5
<210> 27
   <211> 8
   <212> PRT
   <213> Artificial
<220>
   <223> Farnesyl synthetase
<400> 27
   Glu Leu Ala Ser Ala Glu Gln Arg
   1               5
<210> 28
   <211> 11
   <212> PRT
   <213> Artificial
<220>
   <223> Farnesyl synthetase
<400> 28
   Lys Glu Asn Tyr Gly Gln Lys Glu Ala Glu Lys
   1               5                   10
<210> 29
   <211> 11
   <212> PRT
   <213> Artificial
<220>
   <223> Farnesyl synthetase
<400> 29
   Glu Glu Asn Tyr Gly Gln Lys Asp Pro Glu Lys
   1 5 10
<210> 30
   <211> 9
   <212> PRT
   <213> Artificial
<220>
   <223> Farnesyl synthetase
<400> 30
   Asp Glu Asn Tyr Gly Lys Lys Asp Ser
   1 5
<210> 31
   <211> 9
   <212> PRT
   <213> Artificial
<220>
   <223> Farnesyl synthetase
<400> 31
   Arg Val Lys Ala Leu Tyr Glu Glu Leu
   1 5
<210> 32
   <211> 9
   <212> PRT
   <213> Artificial
<220>
   <223> Farnesyl synthetase
<400> 32
   Lys Cys Lys Lys Ile Phe Asn Asp Leu
   1 5
<210> 33
   <211> 10
   <212> PRT
   <213> Artificial
<220>
   <223> Farnesyl synthetase
<400> 33
   Lys Glu Ala Glu Lys Val Ala Arg Val Lys
   1 5 10
<210> 34
   <211> 10
   <212> PRT
   <213> Artificial
<220>
   <223> Farnesyl synthetase
<400> 34
   Lys Asp Ser Val Ala Glu Ala Lys Cys Lys
   1 5 10
<210> 35
   <211> 8
   <212> PRT
   <213> Artificial
<220>
   <223> Farnesyl synthetase
<400> 35
   Lys Asp Pro Glu Lys Val Ala Arg
   1 5
<210> 36
   <211> 9
   <212> PRT
   <213> Artificial
<220>
   <223> Farnesyl synthetase
<400> 36
   Gln Tyr Glu Glu Asp Ser Tyr Ser His
   1 5
<210> 37
   <211> 9
   <212> PRT
   <213> Artificial
<220>
   <223> Farnesyl synthetase
<400> 37
   Lys Tyr Glu Glu Asp Ser Tyr Asn Arg
   1               5
<210> 38
   <211> 9
   <212> PRT
   <213> Artificial
<220>
   <223> Farnesyl synthetase
<400> 38
   Glu Tyr Glu Glu Ser Ile Ala Lys Asp
   1               5
<210> 39
   <211> 8
   <212> PRT
   <213> Artificial
<220>
   <223> Farnesyl synthetase
<400> 39
   Arg Lys Ile Tyr Lys Arg Arg Lys
   1               5
<210> 40
   <211> 8
   <212> PRT
   <213> Artificial
<220>
   <223> Farnesyl synthetase
<400> 40
   Asn Lys Ile Tyr Lys Arg Arg Lys
   1               5
<210> 41
   <211> 8
   <212> PRT
   <213> Artificial
<220>
   <223> Farnesyl synthetase
<400> 41
   Asn Lys Val Tyr Lys Arg Ser Lys
   1               5
<210> 42
   <211> 8
   <212> PRT
   <213> Artificial
<220>
   <223> Hydroxy-methyl-glutaryl coenzyme A reductase
<400> 42
   Ser Gln Asp Glu Val Arg Glu Asn
   1               5
<210> 43
   <211> 8
   <212> PRT
   <213> Artificial
<220>
   <223> Hydroxy-methyl-glutaryl coenzyme A reductase
<400> 43
   Thr Gln Asn Glu Val Val Asp Asn
   1               5
<210> 44
   <211> 10
   <212> PRT
   <213> Artificial
<220>
   <223> Hydroxy-methyl-glutaryl coenzyme A reductase
<400> 44
   Glu Leu Ser Arg Glu Ser Arg Glu Gly Arg
   1               5                   10
<210> 45
   <211> 10
   <212> PRT
   <213> Artificial
<220>
   <223> Hydroxy-methyl-glutaryl coenzyme A reductase
<400> 45
   Glu Leu Ser Asn Ser Asn Lys Tyr Gly Arg
   1               5                   10
<210> 46
   <211> 9
   <212> PRT
   <213> Artificial
<220>
   <223> Hydroxy-methyl-glutaryl coenzyme A reductase
<400> 46
   Arg Val Leu Glu Glu Glu Glu Asn Lys
   1               5
<210> 47
   <211> 10
   <212> PRT
   <213> Artificial
<220>
   <223> Hydroxy-methyl-glutaryl coenzyme A reductase
<400> 47
   Glu Val Leu Glu Glu Glu Glu Asp Arg Lys
   1               5                   10
<210> 48
   <211> 9
   <212> PRT
   <213> Artificial
<220>
   <223> Hydroxy-methyl-glutaryl coenzyme A reductase
<400> 48
   Lys Gly Val Ser Ser Ser Thr Arg Lys
   1               5
<210> 49
   <211> 10
   <212> PRT
   <213> Artificial
<220>
   <223> Hydroxy-methyl-glutaryl coenzyme A reductase
<400> 49
   Ser Val Leu Glu Glu Glu Glu Asp Asn Lys
   1 5 10
<210> 50
   <211> 10
   <212> PRT
   <213> Artificial
<220>
   <223> Hydroxy-methyl-glutaryl coenzyme A reductase
<400> 50
   Asp Glu Asn Val Ser Lys Arg Ile Glu Pro
   1 5 10
<210> 51
   <211> 9
   <212> PRT
   <213> Artificial
<220>
   <223> Hydroxy-methyl-glutaryl coenzyme A reductase
<400> 51
   Asp Asp Met Met Pro Lys Arg Val Glu
   1 5
<210> 52
   <211> 9
   <212> PRT
   <213> Artificial
<220>
   <223> Hydroxy-methyl-glutaryl coenzyme A reductase
<400> 52
   Asp Pro Thr Met Pro Leu Trp Glu Phe
   1 5
<210> 53
   <211> 11
   <212> PRT
   <213> Artificial
<220>
   <223> Hydroxy-methyl-glutaryl coenzyme A reductase
<400> 53
   Lys Lys Val Pro Asp Asn Cys Cys Arg Arg Glu
   1 5 10
<210> 54
   <211> 11
   <212> PRT
   <213> Artificial
<220>
   <223> Hydroxy-methyl-glutaryl coenzyme A reductase
<400> 54
   Lys Lys Ala Pro Asp Asn Cys Cys Arg Arg Glu
   1 5 10
<210> 55
   <211> 11
   <212> PRT
   <213> Artificial
<220>
   <223> Hydroxy-methyl-glutaryl coenzyme A reductase
<400> 55
   Lys Lys Gln Ile Glu Ser Cys Cys Arg Arg Glu
   1 5 10
<210> 56
   <211> 8
   <212> PRT
   <213> Artificial
<220>
   <223> Hydroxy-methyl-glutaryl coenzyme A reductase
<400> 56
   Gln Lys Cys Asp Ser Val Glu Glu
   1 5
<210> 57
   <211> 8
   <212> PRT
   <213> Artificial
<220>
   <223> Hydroxy-methyl-glutaryl coenzyme A reductase
<400> 57
   Glu Lys Leu Ser Ser Val Glu Glu
   1               5
<210> 58
   <211> 7
   <212> PRT
   <213> Artificial
<220>
   <223> Hydroxy-methyl-glutaryl coenzyme A reductase
<400> 58
   His Val Ser Thr Thr Glu Glu
   1               5
<210> 59
   <211> 7
   <212> PRT
   <213> Artificial
<220>
   <223> Hydroxy-methyl-glutaryl coenzyme A reductase
<400> 59
   Glu Thr Leu Ile Ser Asp Gln
   1               5
<210> 60
   <211> 12
   <212> PRT
   <213> Artificial
<220>
   <223> Hydroxy-methyl-glutaryl coenzyme A reductase
<400> 60
   Glu Glu Thr Gly Ile Asn Arg Glu Arg Lys Val Glu
   1               5                   10
<210> 61
   <211> 12
   <212> PRT
   <213> Artificial
<220>
   <223> Hydroxy-methyl-glutaryl coenzyme A reductase
<400> 61
   Glu Glu Pro Gly Val Ser Gln Asp Arg Lys Val Glu
   1               5                   10
<210> 62
   <211> 10
   <212> PRT
   <213> Artificial
<220>
   <223> Hydroxy-methyl-glutaryl coenzyme A reductase
<400> 62
   Glu Ala Ser Ser Lys Glu Glu Thr Glu Ala
   1               5                   10
<210> 63
   <211> 10
   <212> PRT
   <213> Artificial
<220>
   <223> Hydroxy-methyl-glutaryl coenzyme A reductase
<400> 63
   Thr Asp Phe Val Thr Ala Ser Pro Arg Asn
   1               5                   10
<210> 64
   <211> 15
   <212> PRT
   <213> Artificial
<220>
   <223> Hydroxy-methyl-glutaryl coenzyme A reductase
<400> 64
   Glu Pro Glu Ile Glu Leu Pro Arg Glu Pro Arg Pro Asn Glu Glu
   1               5                   10                  15
<210> 65
   <211> 15
   <212> PRT
   <213> Artificial
<220>
   <223> Hydroxy-methyl-glutaryl coenzyme A reductase
<400> 65
   Glu Leu Glu Ile Glu Leu Pro Ser Glu Pro Arg Pro Asn Glu Glu
   1               5                   10                  15
<210> 66
   <211> 15
   <212> PRT
   <213> Artificial
<220>
   <223> Hydroxy-methyl-glutaryl coenzyme A reductase
<400> 66
   Asn Thr Met Phe Asp Leu Pro Glu Glu Pro Arg Pro Leu Asp Glu
   1               5                   10                  15
<210> 67
   <211> 15
   <212> PRT
   <213> Artificial
<220>
   <223> Hydroxy-methyl-glutaryl coenzyme A reductase
<400> 67
   Glu Val Glu Trp Val Leu Glu Thr Glu Leu Lys Ala Pro Arg Pro
   1               5                   10                  15
<210> 68
   <211> 9
   <212> PRT
   <213> Artificial
<220>
   <223> Hydroxy-methyl-glutaryl coenzyme A reductase
<400> 68
   Arg Glu Pro Arg Pro Asn Glu Glu Cys
   1               5
<210> 69
   <211> 8
   <212> PRT
   <213> Artificial
<220>
   <223> Hydroxy-methyl-glutaryl coenzyme A reductase
<400> 69
   Glu Leu Lys Ala Pro Arg Pro Met
   1               5
<210> 70
   <211> 9
   <212> PRT
   <213> Artificial
<220>
   <223> Hydroxy-methyl-glutaryl coenzyme A reductase
<400> 70
   Glu Glu Pro Arg Pro Leu Asp Glu Cys
   1               5
<210> 71
   <211> 15
   <212> PRT
   <213> Artificial
<220>
   <223> Hydroxy-methyl-glutaryl coenzyme A reductase
<220>
   <221> misc_feature
   <222> (5)..(5)
   <223> Xaa can be any naturally occurring amino acid
<400> 71
   His Glu Arg Gly Xaa Ser Ile Arg Arg Gln Leu Leu Ser Lys Lys
   1               5                   10                  15
<210> 72
   <211> 15
   <212> PRT
   <213> Artificial
<220>
   <223> Hydroxy-methyl-glutaryl coenzyme A reductase
<400> 72
   Pro Arg Glu Gly Val Ala Ile Arg Arg Gln Met Leu Ser Asp Lys
   1               5                   10                  15
<210> 73
   <211> 14
   <212> PRT
   <213> Artificial
<220>
   <223> Hydroxy-methyl-glutaryl coenzyme A reductase
<400> 73
   Pro Glu Arg Gly Val Ala Val Arg Arg Gln Ile Ile Ser Lys
   1               5                   10
<210> 74
   <211> 12
   <212> PRT
   <213> Artificial
<220>
   <223> Hydroxy-methyl-glutaryl coenzyme A reductase
<400> 74
   Pro Phe Arg Ala Val Glu Leu Arg Arg Leu Asp Leu
   1               5                   10
<210> 75
   <211> 10
   <212> PRT
   <213> Artificial
<220>
   <223> Hydroxy-methyl-glutaryl coenzyme A reductase
<400> 75
   Leu Gln Tyr Leu Pro Tyr Arg Asp Tyr Asn
   1               5                   10
<210> 76
   <211> 10
   <212> PRT
   <213> Artificial
<220>
   <223> Hydroxy-methyl-glutaryl coenzyme A reductase
<400> 76
   Ile Glu Arg Ile Pro Tyr Lys Asp Tyr Asp
   1               5                   10
<210> 77
   <211> 10
   <212> PRT
   <213> Artificial
<220>
   <223> Hydroxy-methyl-glutaryl coenzyme A reductase
<400> 77
   Leu Gln Ser Leu Pro Tyr Lys Asn Tyr Asn
   1               5                   10
<210> 78
   <211> 10
   <212> PRT
   <213> Artificial
<220>
   <223> Hydroxy-methyl-glutaryl coenzyme A reductase
<400> 78
   Leu Glu Lys Leu Pro Tyr Ala Ser Tyr Asp
   1               5                   10
<210> 79
   <211> 8
   <212> PRT
   <213> Artificial
<220>
   <223> Hydroxy-methyl-glutaryl coenzyme A reductase
<400> 79
   Cys Leu Asp Glu Lys Glu Phe Gln
   1               5
<210> 80
   <211> 8
   <212> PRT
   <213> Artificial
<220>
   <223> Hydroxy-methyl-glutaryl coenzyme A reductase
<400> 80
   Cys Leu Asp Gly Lys Glu Tyr Gln
   1               5
<210> 81
   <211> 8
   <212> PRT
   <213> Artificial
<220>
   <223> Hydroxy-methyl-glutaryl coenzyme A reductase
<400> 81
   Leu Leu Asn Asn Lys Glu Tyr Gln
   1               5
<210> 82
   <211> 8
   <212> PRT
   <213> Artificial
<220>
   <223> Hydroxy-methyl-glutaryl coenzyme A reductase
<400> 82
   Leu Leu Asp Gly Gln Glu Phe Gln
   1               5
<210> 83
   <211> 8
   <212> PRT
   <213> Artificial
<220>
   <223> Hydroxy-methyl-glutaryl coenzyme A reductase
<400> 83
   Leu Leu Asp Gly Arg Ser His Tyr
   1               5
<210> 84
   <211> 12
   <212> PRT
   <213> Artificial
<220>
   <223> Hydroxy-methyl-glutaryl coenzyme A reductase
<400> 84
   Arg Leu Pro Arg Ala Cys Asp Ser Ala Glu Val Lys
   1               5                   10
<210> 85
   <211> 12
   <212> PRT
   <213> Artificial
<220>
   <223> Hydroxy-methyl-glutaryl coenzyme A reductase
<400> 85
   Arg Leu Pro Thr Ala Cys Asp Ala Ala Glu Val Lys
   1 5 10
<210> 86
   <211> 12
   <212> PRT
   <213> Artificial
<220>
   <223> Hydroxy-methyl-glutaryl coenzyme A reductase
<400> 86
   Arg Leu Pro Ser Ala Gln Glu Ala Gly Ala Ile Lys
   1 5 10
<210> 87
   <211> 10
   <212> PRT
   <213> Artificial
<220>
   <223> Hydroxy-methyl-glutaryl coenzyme A reductase
<400> 87
   Glu Lys Ala Leu Ser Lys Leu His Glu Tyr
   1 5 10
<210> 88
   <211> 10
   <212> PRT
   <213> Artificial
<220>
   <223> Hydroxy-methyl-glutaryl coenzyme A reductase
<400> 88
   Glu Lys Ala Leu Val Lys Leu Gln Glu Phe
   1 5 10
<210> 89
   <211> 10
   <212> PRT
   <213> Artificial
<220>
   <223> Hydroxy-methyl-glutaryl coenzyme A reductase
<400> 89
   Glu Gln Ala Leu Ala Arg Leu Gln Glu Glu
   1 5 10
<210> 90
   <211> 10
   <212> PRT
   <213> Artificial
<220>
   <223> Hydroxy-methyl-glutaryl coenzyme A reductase
<400> 90
   Glu Gln Ala Leu His Ala Leu Gln Thr Met
   1 5 10
<210> 91
   <211> 10
   <212> PRT
   <213> Artificial
<220>
   <223> Hydroxy-methyl-glutaryl coenzyme A reductase
<400> 91
   Cys Lys Asp Asn Pro Gly Glu Asn Ala Arg
   1 5 10
<210> 92
   <211> 10
   <212> PRT
   <213> Artificial
<220>
   <223> Hydroxy-methyl-glutaryl coenzyme A reductase
<400> 92
   Ser Thr Glu Thr Pro Gly Lys Asn Ala Cys
   1               5                   10
<210> 93
   <211> 10
   <212> PRT
   <213> Artificial
<220>
   <223> Hydroxy-methyl-glutaryl coenzyme A reductase
<400> 93
   Asn Ile His Gly Ser Gly Leu Asn Ala Ser
   1               5                   10
<210> 94
   <211> 10
   <212> PRT
   <213> Artificial
<220>
   <223> Hydroxy-methyl-glutaryl coenzyme A reductase
<400> 94
   His Asn Arg Ser Lys Ile Asn Leu Gln Asp
   1               5                   10
<210> 95
   <211> 10
   <212> PRT
   <213> Artificial
<220>
   <223> Hydroxy-methyl-glutaryl coenzyme A reductase
<400> 95
   His Asn Arg Ser Ala Leu Asn Ile Asp Glu
   1               5                   10
<210> 96
   <211> 8
   <212> PRT
   <213> Artificial

<220>
   <223> Glucagon precursor
<400> 96
   Asp Tyr Ser Lys Tyr Leu Asp Ser
   1 5
<210> 97
   <211> 7
   <212> PRT
   <213> Artificial
<220>
   <223> Glucagon precursor
<400> 97
   Asp Ser Arg Arg Ala Gln Asp
   1 5
<210> 98
   <211> 13
   <212> PRT
   <213> Artificial
<220>
   <223> Glucagon precursor
<400> 98
   Asp Tyr Ser Lys Tyr Leu Asp Ser Arg Arg Ala Gln Asp
   1 5 10
<210> 99
   <211> 13
   <212> PRT
   <213> Artificial
<220>
   <223> Glucagon precursor
<400> 99
   Asp Val Ala Ser Leu Thr Asp Tyr Leu Lys Ser Lys Arg
   1 5 10
<210> 100
   <211> 13
   <212> PRT
   <213> Artificial
<220>
   <223> Glucagon precursor
<400> 100
   Asp Tyr Ser Lys Tyr Met Asp Asn Arg Arg Ala Lys Asp
   1               5                   10
<210> 101
   <211> 13
   <212> PRT
   <213> Artificial
<220>
   <223> Glucagon precursor
<400> 101
   Asp Tyr Ser Lys Tyr Leu Asp Asn Arg Arg Ala Lys Asp
   1               5                   10
<210> 102
   <211> 13
   <212> PRT
   <213> Artificial
<220>
   <223> Glucagon precursor
<400> 102
   Asp Tyr Ser Lys Tyr Leu Asp Ser Arg Arg Ala Gln Gln
   1               5                   10
<210> 103
   <211> 8
   <212> PRT
   <213> Artificial
<220>
   <223> Glucagon precursor
<400> 103
   Asp Tyr Ser Lys Tyr Leu Asp Ser
   1               5
<210> 104
   <211> 7
   <212> PRT
   <213> Artificial
<220>
   <223> Glucagon precursor
<400> 104
   Asp Ser Arg Arg Ala Gln Asp
   1 5
<210> 105
   <211> 13
   <212> PRT
   <213> Artificial
<220>
   <223> Glucagon precursor
<400> 105
   Asp Tyr Ser Lys Tyr Leu Asp Ser Arg Arg Ala Gln Asp
   1 5 10
<210> 106
   <211> 13
   <212> PRT
   <213> Artificial
<220>
   <223> Glucagon precursor
<400> 106
   Asp Val Ala Ser Leu Thr Asp Tyr Leu Lys Ser Lys Arg
   1 5 10
<210> 107
   <211> 13
   <212> PRT
   <213> Artificial
<220>
   <223> Glucagon precursor
<400> 107
   Asp Tyr Ser Lys Tyr Met Asp Asn Arg Arg Ala Lys Asp
   1 5 10
<210> 108
   <211> 13
   <212> PRT
   <213> Artificial
<220>
   <223> Glucagon precursor
<400> 108
   Asp Tyr Ser Lys Tyr Leu Asp Asn Arg Arg Ala Lys Asp
   1 5 10
<210> 109
   <211> 13
   <212> PRT
   <213> Artificial
<220>
   <223> Glucagon precursor
<400> 109
   Asp Tyr Ser Lys Tyr Leu Asp Ser Arg Arg Ala Gln Gln
   1 5 10
<210> 110
   <211> 13
   <212> PRT
   <213> Artificial
<220>
   <223> Glucagon precursor
<400> 110
   Asp Tyr Ser Lys Phe Leu Asp Thr Arg Arg Ala Gln Asp
   1 5 10
<210> 111
   <211> 13
   <212> PRT
   <213> Artificial
<220>
   <223> Glucagon precursor
<400> 111
   Asp Tyr Ser Lys Tyr Gln Glu Glu Arg Met Ala Gln Asp
   1 5 10
<210> 112
   <211> 13
   <212> PRT
   <213> Artificial
<220>
   <223> Glucagon precursor
<400> 112
   Asp Tyr Ser Lys Tyr Leu Glu Thr Arg Arg Ala Gln Asp
   1 5 10
<210> 113
   <211> 13
   <212> PRT
   <213> Artificial
<220>
   <223> Glucagon precursor
<400> 113
   Asp Tyr Ser Lys Tyr Leu Asp Asn Arg Arg Thr Lys Asp
   1 5 10
<210> 114
   <211> 13
   <212> PRT
   <213> Artificial
<220>
   <223> Glucagon precursor
<400> 114
   Asp Met Ser Ser Tyr Leu Glu Glu Lys Ala Ala Lys Glu
   1 5 10
<210> 115
   <211> 13
   <212> PRT
   <213> Artificial
<220>
   <223> Glucagon precursor
<400> 115
   Asp Phe Asn Lys Ala Leu Asp Ile Lys Ala Ala Gln Glu
   1 5 10
<210> 116
   <211> 9
   <212> PRT
   <213> Artificial
<220>
   <223> Glucagon precursor
<400> 116
   Asp Pro Leu Ser Asp Pro Asp Gln Met
   1               5
<210> 117
   <211> 8
   <212> PRT
   <213> Artificial
<220>
   <223> Glucagon precursor
<400> 117
   Asn Glu Asp Lys Arg His Ser Gln
   1               5
<210> 118
   <211> 15
   <212> PRT
   <213> Artificial
<220>
   <223> Glucagon precursor
<400> 118
   Lys Asp Glu Pro Arg Glu Leu Ser Asn Met Lys Arg His Ser Glu
   1               5                   10                  15
<210> 119
   <211> 9
   <212> PRT
   <213> Artificial
<220>
   <223> Glucagon precursor
<400> 119
   Arg Glu Leu Ser Asn Met Lys Arg His
   1               5
<210> 120
   <211> 13
   <212> PRT
   <213> Artificial
<220>
   <223> Glucagon precursor
<220>
   <221> misc_feature
   <222> (10)..(10)
   <223> Xaa can be any naturally occurring amino acid
<400> 120
   Glu Ala Arg Glu Leu Ser Thr Pro Lys Xaa His Ser Glu
   1               5                   10
<210> 121
   <211> 12
   <212> PRT
   <213> Artificial
<220>
   <223> Glucagon precursor
<400> 121
   Glu Asp Pro Asp Gln Ile Asn Glu Asp Lys Arg His
   1               5                   10
<210> 122
   <211> 14
   <212> PRT
   <213> Artificial
<220>
   <223> Glucagon precursor
<400> 122
   Asp Asp Pro Asp Gln Met Asn Glu Asp Lys Arg His Ser Gln
   1               5                   10
<210> 123
   <211> 14
   <212> PRT
   <213> Artificial
<220>
   <223> Glucagon precursor
<400> 123
   Gly Asp Pro Asp Gln Ile Asn Glu Asp Lys Arg His Ser Gln
   1               5                   10
<210> 124
   <211> 14
   <212> PRT
   <213> Artificial
<220>
   <223> Glucagon precursor
<400> 124
   Asp Glu Ser Arg Gln Leu Asn Glu Val Lys Arg His Ser Gln
   1 5 10
<210> 125
   <211> 9
   <212> PRT
   <213> Artificial
<220>
   <223> Glucagon precursor
<400> 125
   Met Asn Thr Lys Arg Asn Arg Asn Asn
   1 5
<210> 126
   <211> 9
   <212> PRT
   <213> Artificial
<220>
   <223> Glucagon precursor
<400> 126
   Lys Arg His Asp Glu Phe Glu Arg His
   1 5
<210> 127
   <211> 20
   <212> PRT
   <213> Artificial
<220>
   <223> Glucagon precursor
<400> 127
   Met Asn Thr Lys Arg Asn Arg Asn Arg Asn Asn Lys Arg His Asp Glu
   1 5 10 15
   Phe Glu Arg His
   20
<210> 128
   <211> 10
   <212> PRT
   <213> Artificial
<220>
   <223> Glucagon precursor
<400> 128
   Asn Lys Arg Ser Gly Val Ala Glu Lys Arg
   1               5                   10
<210> 129
   <211> 11
   <212> PRT
   <213> Artificial
<220>
   <223> Glucagon precursor
<400> 129
   Val Lys Arg Asn Arg Asn Asn Ile Ala Lys Arg
   1               5                   10
<210> 130
   <211> 9
   <212> PRT
   <213> Artificial
<220>
   <223> Glucagon precursor
<400> 130
   Lys Asn Thr Lys Arg Asn Arg Asn Glu
   1               5
<210> 131
   <211> 12
   <212> PRT
   <213> Artificial
<220>
   <223> Glucagon precursor
<220>
   <221> misc_feature
   <222> (6)..(7)
   <223> Xaa can be any naturally occurring amino acid
<400> 131
   Thr Lys Arg Asn Gly Xaa Xaa Gly Gln Glu Asp Lys
   1               5                   10
<210> 132
   <211> 9
   <212> PRT
   <213> Artificial
<220>
   <223> Glucagon precursor
<400> 132
   Gln Glu Asp Lys Glu Asn Asp Lys Phe
   1 5
<210> 133
   <211> 11
   <212> PRT
   <213> Artificial
<220>
   <223> Glucagon precursor
<400> 133
   Lys Arg His Ser Glu Phe Glu Arg His Ala Glu
   1 5 10
<210> 134
   <211> 8
   <212> PRT
   <213> Artificial
<220>
   <223> Glucagon precursor
<400> 134
   Gln Asp Thr Glu Glu Lys Ser Arg
   1 5
<210> 135
   <211> 8
   <212> PRT
   <213> Artificial
<220>
   <223> Glucagon precursor
<400> 135
   Gln Asp Thr Glu Glu Lys Pro Arg
   1 5
<210> 136
   <211> 8
   <212> PRT
   <213> Artificial
<220>
   <223> Glucagon precursor
<400> 136
   Gln Asp Thr Glu Glu Asn Ala Arg
   1               5
<210> 137
   <211> 9
   <212> PRT
   <213> Artificial
<220>
   <223> Glucagon precursor
<400> 137
   Asp Gln Asp Pro Asp Arg Asn Ser Met
   1               5
<210> 138
   <211> 11
   <212> PRT
   <213> Artificial
<220>
   <223> Glucagon precursor
<400> 138
   Lys Gly Arg Gly Arg Arg Asp Phe Pro Glu Glu
   1               5                   10
<210> 139
   <211> 10
   <212> PRT
   <213> Artificial
<220>
   <223> Glucagon precursor
<400> 139
   Asp Arg Leu Lys Ala Gln Val Arg Arg Glu
   1               5                   10
<210> 140
   <211> 8
   <212> PRT
   <213> Artificial
<220>
   <223> Glucagon precursor
<400> 140
   Ala Lys Leu Lys Ser Gly Lys Val
   1               5
<210> 141
   <211> 8
   <212> PRT
   <213> Artificial
<220>
   <223> Glucagon precursor
<400> 141
   Lys Ser Gly Gln Pro Lys Pro Glu
   1               5
<210> 142
   <211> 8
   <212> PRT
   <213> Artificial
<220>
   <223> Glucagon precursor
<400> 142
   Lys Gln Gly Gln Asp Arg Arg Glu
   1               5
<210> 143
   <211> 7
   <212> PRT
   <213> Artificial
<220>
   <223> Gastrin precursor
<400> 143
   Ala Asp Pro Ser Lys Lys Gln
   1               5
<210> 144
   <211> 8
   <212> PRT
   <213> Artificial
<220>
   <223> Gastrin precursor
<400> 144
   Arg Thr Asp Leu Ser Lys Lys Gln
   1 5
<210> 145
   <211> 7
   <212> PRT
   <213> Artificial
<220>
   <223> Gastrin precursor
<400> 145
   Ala Asp Leu Ser Lys Lys Gln
   1 5
<210> 146
   <211> 7
   <212> PRT
   <213> Artificial
<220>
   <223> Gastrin precursor
<400> 146
   Asp Leu Ser Lys Lys Gln Arg
   1 5
<210> 147
   <211> 8
   <212> PRT
   <213> Artificial
<220>
   <223> Gastrin precursor
<400> 147
   Trp Leu Glu Glu Glu Glu Glu Ala
   1 5
<210> 148
   <211> 7
   <212> PRT
   <213> Artificial
<220>
   <223> Gastrin precursor
<400> 148
   Trp Met Glu Glu Glu Glu Ala
   1               5
<210> 149
   <211> 7
   <212> PRT
   <213> Artificial
<220>
   <223> Gastrin precursor
<400> 149
   Trp Val Glu Glu Glu Glu Ala
   1               5
<210> 150
   <211> 7
   <212> PRT
   <213> Artificial
<220>
   <223> Gastrin precursor
<400> 150
   Trp Ala Glu Glu Glu Glu Ala
   1               5
<210> 151
   <211> 8
   <212> PRT
   <213> Artificial
<220>
   <223> Gastrin precursor
<400> 151
   Pro Met Glu Glu Glu Glu Glu Ala
   1               5
<210> 152
   <211> 11
   <212> PRT
   <213> Artificial
<220>
   <223> Gastrin precursor
<400> 152
   Asp Phe Gly Arg Arg Ser Ala Glu Asp Glu Asn
   1 5 10
<210> 153
   <211> 7
   <212> PRT
   <213> Artificial
<220>
   <223> Gastrin precursor
<400> 153
   Met Asp Phe Gly Arg Arg Ser
   1 5
<210> 154
   <211> 7
   <212> PRT
   <213> Artificial
<220>
   <223> Gastrin precursor
<400> 154
   Arg Arg Ser Ala Glu Asp Glu
   1 5
<210> 155
   <211> 11
   <212> PRT
   <213> Artificial
<220>
   <223> Gastrin precursor
<400> 155
   Arg Arg Ser Ala Glu Asp Gly Asp Gln His Pro
   1 5 10
<210> 156
   <211> 9
   <212> PRT
   <213> Artificial
<220>
   <223> Gastrin precursor
<400> 156
   Arg Asp Leu Glu Leu Pro Trp Leu Glu
   1 5
<210> 157
   <211> 10
   <212> PRT
   <213> Artificial
<220>
   <223> Gastrin precursor
<400> 157
   Arg Gly Lys Glu Pro His Glu Leu Asp Arg
   1 5 10
<210> 158
   <211> 7
   <212> PRT
   <213> Artificial
<220>
   <223> Gastrin precursor
<400> 158
   Arg Gly Gln Glu Pro Leu Arg
   1 5
<210> 159
   <211> 7
   <212> PRT
   <213> Artificial
<220>
   <223> Gastrin precursor
<400> 159
   Glu Pro His Trp Leu Asn Arg
   1 5
<210> 160
   <211> 8
   <212> PRT
   <213> Artificial
<220>
   <223> Gastrin precursor
<400> 160
   Lys Pro Arg Ser Gln Gln Pro Asp
   1 5
<210> 161
   <211> 8
   <212> PRT
   <213> Artificial
<220>
   <223> Gastrin precursor
<400> 161
   Lys Pro Gly Phe Gln Leu Gln Asp
   1 5
<210> 162
   <211> 8
   <212> PRT
   <213> Artificial
<220>
   <223> Gastrin precursor
<400> 162
   Lys Pro His Ser His Leu Gln Asp
   1 5
<210> 163
   <211> 8
   <212> PRT
   <213> Artificial
<220>
   <223> Gastrin precursor
<400> 163
   Lys Pro Arg Ser Gln Leu Gln Asp
   1 5
<210> 164
   <211> 12
   <212> PRT
   <213> Artificial
<220>
   <223> Gonadoliberin precursor
<400> 164
   Gln His Trp Ser Tyr Gly Leu Arg Pro Gly Gly Lys
   1               5                   10
<210> 165
   <211> 9
   <212> PRT
   <213> Artificial
<220>
   <223> Gonadoliberin precursor
<400> 165
   Gly Leu Gln Pro Gly Gly Lys Arg Asp
   1               5
<210> 166
   <211> 10
   <212> PRT
   <213> Artificial
<220>
   <223> Gonadoliberin precursor
<400> 166
   His Gly Trp Leu Pro Gly Gly Lys Arg Asp
   1               5                   10
<210> 167
   <211> 9
   <212> PRT
   <213> Artificial
<220>
   <223> Gonadoliberin precursor
<400> 167
   Gly Trp Leu Pro Gly Gly Lys Arg Asp
   1               5
<210> 168
   <211> 10
   <212> PRT
   <213> Artificial
<220>
   <223> Gonadoliberin precursor
<400> 168
   His Gly Trp Tyr Pro Gly Gly Lys Arg Asp
   1 5 10
<210> 169
   <211> 10
   <212> PRT
   <213> Artificial
<220>
   <223> Gonadoliberin precursor
<400> 169
   Gln His Tyr Ser Leu Glu Trp Lys Pro Gly
   1 5 10
<210> 170
   <211> 9
   <212> PRT
   <213> Artificial
<220>
   <223> Gonadoliberin precursor
<400> 170
   Arg Pro Gly Gly Lys Arg Asp Ala Glu
   1 5
<210> 171
   <211> 10
   <212> PRT
   <213> Artificial
<220>
   <223> Gonadoliberin precursor
<400> 171
   Arg Pro Gly Gly Lys Arg Asn Thr Glu His
   1 5 10
<210> 172
   <211> 9
   <212> PRT
   <213> Artificial
<220>
   <223> Gonadoliberin precursor
<400> 172
   Gln Pro Gly Gly Lys Arg Asp Ala Glu
   1               5
<210> 173
   <211> 6
   <212> PRT
   <213> Artificial
<220>
   <223> Plasminogen activator inhibitor 1
<400> 173
   Gln Ala Ser Lys Asp Arg
   1               5
<210> 174
   <211> 6
   <212> PRT
   <213> Artificial
<220>
   <223> Plasminogen activator inhibitor 1
<400> 174
   Lys Ile Asp Asp Lys Gly
   1               5
<210> 175
   <211> 5
   <212> PRT
   <213> Artificial
<220>
   <223> Plasminogen activator inhibitor 1
<400> 175
   Trp Asn Lys Asp Glu
   1               5
<210> 176
   <211> 6
   <212> PRT
   <213> Artificial
<220>
   <223> Plasminogen activator inhibitor 1
<400> 176
   Glu Val Glu Arg Ala Arg
   1 5
<210> 177
   <211> 9
   <212> PRT
   <213> Artificial
<220>
   <223> Plasminogen activator inhibitor 1
<400> 177
   His Arg Arg Leu Phe His Lys Ser Asp
   1 5
<210> 178
   <211> 7
   <212> PRT
   <213> Artificial
<220>
   <223> Plasminogen activator inhibitor 1
<400> 178
   Lys Thr Pro Phe Pro Asp Ser
   1 5
<210> 179
   <211> 6
   <212> PRT
   <213> Artificial
<220>
   <223> Plasminogen activator inhibitor 1
<400> 179
   Tyr Glu Lys Glu Val Pro
   1 5
<210> 180
   <211> 7
   <212> PRT
   <213> Artificial
<220>
   <223> Plasminogen activator inhibitor 1
<400> 180
   Glu Val Asp Leu Arg Lys Pro
   1 5
<210> 181
   <211> 8
   <212> PRT
   <213> Artificial
<220>
   <223> Plasminogen activator inhibitor 1
<400> 181
   Lys Val Lys Ile Glu Val Asn Glu
   1 5
<210> 182
   <211> 7
   <212> PRT
   <213> Artificial
<220>
   <223> Plasminogen activator inhibitor 1
<400> 182
   Ala Arg Met Ala Pro Glu Glu
   1 5
<210> 183
   <211> 6
   <212> PRT
   <213> Artificial
<220>
   <223> Plasminogen activator inhibitor 1
<400> 183
   Arg Ala Ser Lys Asp Arg
   1 5
<210> 184
   <211> 6
   <212> PRT
   <213> Artificial
<220>
   <223> Plasminogen activator inhibitor 1
<400> 184
   Lys Ile Glu Glu Lys Gly
   1 5
<210> 185
   <211> 6
   <212> PRT
   <213> Artificial
<220>
   <223> Plasminogen activator inhibitor 1
<400> 185
   Lys Val Asn Glu Lys Gly
   1 5
<210> 186
   <211> 6
   <212> PRT
   <213> Artificial
<220>
   <223> Plasminogen activator inhibitor 1
<400> 186
   Asn Ile Ser Glu Arg Gly
   1 5
<210> 187
   <211> 5
   <212> PRT
   <213> Artificial
<220>
   <223> Plasminogen activator inhibitor 1
<400> 187
   Trp Asn Lys Asn Glu
   1 5
<210> 188
   <211> 7
   <212> PRT
   <213> Artificial
<220>
   <223> Plasminogen activator inhibitor 1
<400> 188
   Lys Met Pro Phe Pro Glu Ser
   1               5
<210> 189
   <211> 7
   <212> PRT
   <213> Artificial
<220>
   <223> Plasminogen activator inhibitor 1
<400> 189
   Lys Thr Pro Phe Leu Glu Ala
   1               5
<210> 190
   <211> 9
   <212> PRT
   <213> Artificial
<220>
   <223> Plasminogen activator inhibitor 1
<400> 190
   His His Arg Leu Phe His Lys Ser Asp
   1               5
<210> 191
   <211> 9
   <212> PRT
   <213> Artificial
<220>
   <223> Plasminogen activator inhibitor 1
<400> 191
   His Gln Arg Leu Phe His Lys Ser Asp
   1               5
<210> 192
   <211> 10
   <212> PRT
   <213> Artificial
<220>
   <223> Plasminogen activator inhibitor 1
<400> 192
   Glu Val Asp Leu Arg Gly Pro Leu Glu Lys
   1 5 10
<210> 193
   <211> 9
   <212> PRT
   <213> Artificial
<220>
   <223> Plasminogen activator inhibitor 1
<400> 193
   Glu Ile Asp Leu Arg Arg Pro Leu Glu
   1 5
<210> 194
   <211> 8
   <212> PRT
   <213> Artificial
<220>
   <223> Plasminogen activator inhibitor 1
<400> 194
   Ala Arg Met Ala Pro Thr Glu Met
   1 5
<210> 195
   <211> 8
   <212> PRT
   <213> Artificial
<220>
   <223> Plasminogen activator inhibitor 2
<400> 195
   Lys Trp Lys Thr Pro Phe Glu Lys
   1 5
<210> 196
   <211> 13
   <212> PRT
   <213> Artificial
<220>
   <223> Plasminogen activator inhibitor 2
<400> 196
   Arg Glu Lys Leu Asn Ile Gly Tyr Ile Glu Asp Leu Lys
   1 5 10
<210> 197
   <211> 13
   <212> PRT
   <213> Artificial
<220>
   <223> Plasminogen activator inhibitor 2
<400> 197
   His Ala Lys Leu Asn Ile Gly Tyr Ile Lys Asp Leu Lys
   1 5 10
<210> 198
   <211> 10
   <212> PRT
   <213> Artificial
<220>
   <223> Plasminogen activator inhibitor 2
<400> 198
   Lys Asp Lys Met Ala Glu Asp Glu Val Glu
   1 5 10
<210> 199
   <211> 8
   <212> PRT
   <213> Artificial
<220>
   <223> Plasminogen activator inhibitor 2
<400> 199
   Lys Asp Thr Leu Asp Glu Asp Asp
   1 5
<210> 200
   <211> 9
   <212> PRT
   <213> Artificial
<220>
   <223> Plasminogen activator inhibitor 2
<400> 200
   Lys Leu Glu Glu His Tyr Glu Leu Arg
   1 5
<210> 201
   <211> 9
   <212> PRT
   <213> Artificial
<220>
   <223> Plasminogen activator inhibitor 2
<400> 201
   Lys Leu Ala Gln Ser Tyr Glu Leu Lys
   1 5
<210> 202
   <211> 9
   <212> PRT
   <213> Artificial
<220>
   <223> Plasminogen activator inhibitor 2
<400> 202
   Glu Arg Asn Asp Leu Phe Leu Ser Glu
   1 5
<210> 203
   <211> 8
   <212> PRT
   <213> Artificial
<220>
   <223> Plasminogen activator inhibitor 2
<400> 203
   Asp Val Asn Glu Glu Gly Thr Glu
   1 5
<210> 204
   <211> 8
   <212> PRT
   <213> Artificial
<220>
   <223> Plasminogen activator inhibitor 2
<400> 204
   Asp Val Thr Glu Glu Gly Thr Val
   1               5
<210> 205
   <211> 8
   <212> PRT
   <213> Artificial
<220>
   <223> Plasminogen activator inhibitor 2
<400> 205
   Arg Gly Ser Thr Glu Asp Glu Gln
   1               5
<210> 206
   <211> 7
   <212> PRT
   <213> Artificial
<220>
   <223> Plasminogen activator inhibitor 2
<400> 206
   Gly Gly Asn Thr Glu Gln Gln
   1               5
<210> 207
   <211> 9
   <212> PRT
   <213> Artificial
<220>
   <223> Plasminogen activator inhibitor 2
<400> 207
   Glu Lys Ser Ala Ser Phe Arg Glu Glu
   1               5
<210> 208
   <211> 9
   <212> PRT
   <213> Artificial
<220>
   <223> Plasminogen activator inhibitor 2
<400> 208
   Glu Lys Ser Ala Arg Phe Lys Glu Glu
   1               5
<210> 209
   <211> 6
   <212> PRT
   <213> Artificial
<220>
   <223> Plasminogen activator inhibitor 2
<400> 209
   Glu Glu Ala Arg Lys Lys
   1               5
<210> 210
   <211> 6
   <212> PRT
   <213> Artificial
<220>
   <223> Plasminogen activator inhibitor 2
<400> 210
   Glu Glu Ala Arg Glu Lys
   1               5
<210> 211
   <211> 7
   <212> PRT
   <213> Artificial
<220>
   <223> Plasminogen activator inhibitor 2
<400> 211
   Lys Thr Gln Thr Lys Gly Lys
   1               5
<210> 212
   <211> 7
   <212> PRT
   <213> Artificial
<220>
   <223> Plasminogen activator inhibitor 2
<400> 212
   Lys Thr Gln Thr Lys Gly Glu
   1 5
<210> 213
   <211> 9
   <212> PRT
   <213> Artificial
<220>
   <223> Plasminogen activator inhibitor 2
<400> 213
   Glu Gly Ser Val Asp Gly Asp Thr Arg
   1 5
<210> 214
   <211> 9
   <212> PRT
   <213> Artificial
<220>
   <223> Plasminogen activator inhibitor 2
<400> 214
   Glu Gly Ser Val Asp Glu Asp Thr Lys
   1 5
<210> 215
   <211> 9
   <212> PRT
   <213> Artificial
<220>
   <223> Alzheimer amyloid A4
<400> 215
   Asp Ser Ala Asp Ala Glu Glu Asp Asp
   1 5
<210> 216
   <211> 9
   <212> PRT
   <213> Artificial
<220>
   <223> Alzheimer amyloid A4
<400> 216
   Glu Glu Glu Glu Val Ala Glu Val Glu
   1 5
<210> 217
   <211> 8
   <212> PRT
   <213> Artificial
<220>
   <223> Alzheimer amyloid A4
<400> 217
   Glu Glu Glu Glu Ala Asp Asp Asp
   1 5
<210> 218
   <211> 10
   <212> PRT
   <213> Artificial
<220>
   <223> Alzheimer amyloid A4
<400> 218
   Ala Asp Asp Asp Glu Asp Asp Glu Asp Gly
   1 5 10
<210> 219
   <211> 8
   <212> PRT
   <213> Artificial
<220>
   <223> Alzheimer amyloid A4
<400> 219
   Asp Glu Asp Gly Asp Glu Val Glu
   1 5
<210> 220
   <211> 9
   <212> PRT
   <213> Artificial
<220>
   <223> Alzheimer amyloid A4
<400> 220
   Glu Glu Ala Glu Glu Pro Tyr Glu Glu
   1 5
<210> 221
   <211> 15
   <212> PRT
   <213> Artificial
<220>
   <223> Alzheimer amyloid A4
<400> 221
   Ala Asp Val Glu Glu Glu Glu Ala Asp Asp Asp Glu Asp Val Glu
   1 5 10 15
<210> 222
   <211> 7
   <212> PRT
   <213> Artificial
<220>
   <223> Alzheimer amyloid A4
<400> 222
   Lys Ala Lys Glu Arg Leu Glu
   1 5
<210> 223
   <211> 7
   <212> PRT
   <213> Artificial
<220>
   <223> Alzheimer amyloid A4
<400> 223
   Glu Ala Lys His Arg Glu Arg
   1 5
<210> 224
   <211> 8
   <212> PRT
   <213> Artificial
<220>
   <223> Alzheimer amyloid A4
<400> 224
   Arg Glu Trp Glu Glu Ala Glu Arg
   1               5
<210> 225
   <211> 7
   <212> PRT
   <213> Artificial
<220>
   <223> Alzheimer amyloid A4
<400> 225
   Arg Ala Glu Gln Lys Asp Arg
   1               5
<210> 226
   <211> 9
   <212> PRT
   <213> Artificial
<220>
   <223> Apolipoprotein E
<400> 226
   Glu Pro Glu Thr Glu Pro Asp Val Arg
   1               5
<210> 227
   <211> 8
   <212> PRT
   <213> Artificial
<220>
   <223> Apolipoprotein E
<400> 227
   Glu Gly Glu Pro Glu Val Thr Asp
   1               5
<210> 228
   <211> 9
   <212> PRT
   <213> Artificial
<220>
   <223> Apolipoprotein E
<400> 228
   Glu Thr Glu Pro Glu Pro Glu Leu Arg
   1 5
<210> 229
   <211> 6
   <212> PRT
   <213> Artificial
<220>
   <223> Apolipoprotein E
<400> 229
   Glu Asp Val Cys Gly Arg
   1 5
<210> 230
   <211> 6
   <212> PRT
   <213> Artificial
<220>
   <223> Apolipoprotein E
<400> 230
   Glu Asp Val Arg Gly Arg
   1 5
<210> 231
   <211> 6
   <212> PRT
   <213> Artificial
<220>
   <223> Apolipoprotein E
<400> 231
   Glu Asp Leu Arg Asn Arg
   1 5
<210> 232
   <211> 8
   <212> PRT
   <213> Artificial
<220>
   <223> Apolipoprotein E
<400> 232
   His Leu Arg Lys Leu Arg Lys Arg
   1 5
<210> 233
   <211> 8
   <212> PRT
   <213> Artificial
<220>
   <223> Apolipoprotein E
<400> 233
   His Pro Arg Lys Met Lys Arg Arg
   1 5
<210> 234
   <211> 8
   <212> PRT
   <213> Artificial
<220>
   <223> Apolipoprotein E
<400> 234
   His Leu Arg Lys Met Arg Lys Arg
   1 5
<210> 235
   <211> 9
   <212> PRT
   <213> Artificial
<220>
   <223> Apolipoprotein E
<400> 235
   Arg Asp Arg Leu Asp Glu Val Lys Glu
   1 5
<210> 236
   <211> 6
   <212> PRT
   <213> Artificial
<220>
   <223> Apolipoprotein E
<400> 236
   Arg Ala Arg Met Glu Glu
   1               5
<210> 237
   <211> 6
   <212> PRT
   <213> Artificial
<220>
   <223> Apolipoprotein E
<400> 237
   Arg Gly Arg Met Glu Glu
   1               5
<210> 238
   <211> 6
   <212> PRT
   <213> Artificial
<220>
   <223> Apolipoprotein E
<400> 238
   Arg Gly Arg Leu Glu Glu
   1               5
<210> 239
   <211> 13
   <212> PRT
   <213> Artificial
<220>
   <223> Herpes virus 1 (HSV1) glycoprotein B
<400> 239
   Asp Tyr Thr Glu Val Gln Arg Arg Asn Gln Leu His Asp
   1               5                   10
<210> 240
   <211> 10
   <212> PRT
   <213> Artificial
<220>
   <223> Herpes virus 1 (HSV1) glycoprotein B
<400> 240
   Lys Glu Leu Lys Asn Pro Thr Asn Pro Asp
   1 5 10
<210> 241
   <211> 12
   <212> PRT
   <213> Artificial

<220>
   <223> Herpes virus 1 (HSV1) glycoprotein B
<400> 241
   Glu Gly Glu Glu Gly Gly Asp Phe Asp Glu Ala Lys
   1 5 10
<210> 242
   <211> 13
   <212> PRT
   <213> Artificial
<220>
   <223> Herpes virus 1 (HSV1) glycoprotein B
<400> 242
   Asp Glu Ala Lys Leu Ala Glu Ala Arg Glu Met Ile Arg
   1 5 10
<210> 243
   <211> 9
   <212> PRT
   <213> Artificial
<220>
   <223> Herpes virus 1 (HSV1) glycoprotein B
<400> 243
   Asp Met Val Met Arg Lys Arg Arg Asn
   1 5
<210> 244
   <211> 21
   <212> PRT
   <213> Artificial
<220>
   <223> Herpes virus 1 (HSV1) glycoprotein B
<400> 244
   Arg Lys Arg Arg Asn Thr Asn Tyr Thr Gln Val Pro Asn Lys Asp Ala
   1               5                   10                  15
   Asp Glu Asp Asp Leu
               20
<210> 245
   <211> 9
   <212> PRT
   <213> Artificial
<220>
   <223> Herpes virus 2 (HSV 23, 2H) glycoprotein B
<400> 245
   Lys Ala Arg Lys Arg Lys Thr Lys Lys
   1               5
<210> 246
   <211> 8
   <212> PRT
   <213> Artificial
<220>
   <223> Herpes virus 2 (HSV 23, 2H) glycoprotein B
<400> 246
   Lys Lys Pro Pro Lys Arg Pro Glu
   1               5
<210> 247
   <211> 9
   <212> PRT
   <213> Artificial
<220>
   <223> Herpes virus 2 (HSV 23, 2H) glycoprotein B
<400> 247
   Arg Asp Ala Arg Glu Ala Ile Asp Arg
   1               5
<210> 248
   <211> 10
   <212> PRT
   <213> Artificial
<220>
   <223> Herpes virus 2 (HSV 23, 2H) glycoprotein B
<400> 248
   Arg Glu Tyr Met Arg Glu Gln Asp Arg Lys
   1 5 10
<210> 249
   <211> 7
   <212> PRT
   <213> Artificial
<220>
   <223> Herpes virus 2 (HSV 23, 2H) glycoprotein B
<400> 249
   Arg Lys Arg Asn Lys Ala Arg
   1 5
<210> 250
   <211> 10
   <212> PRT
   <213> Artificial
<220>
   <223> Herpes virus 2 (HSV 23, 2H) glycoprotein B
<400> 250
   Glu Asp Glu Ala Gly Asp Glu Asp Glu Leu
   1 5 10
<210> 251
   <211> 23
   <212> PRT
   <213> Artificial
<220>
   <223> Herpes virus 2 (HSV 23, 2H) glycoprotein B
<400> 251
   Arg Lys Arg Asn Lys Ala Arg Tyr Ser Pro Leu His Asn Glu Asp Glu
   1 5 10 15
   Ala Gly Asp Glu Asp Glu Leu
   20
<210> 252
   <211> 12
   <212> PRT
   <213> Artificial
<220>
   <223> Treponema pallidum membrane protein TMPA
<400> 252
   Lys Glu Glu Ala Glu Lys Lys Ala Ala Glu Gln Arg
   1 5 10
<210> 253
   <211> 8
   <212> PRT
   <213> Artificial
<220>
   <223> Treponema pallidum membrane protein TMPA
<400> 253
   His Ala Asp Arg Arg Leu Met Glu
   1 5
<210> 254
   <211> 10
   <212> PRT
   <213> Artificial
<220>
   <223> Treponema pallidum membrane protein TMPA
<400> 254
   Asp Val Glu Arg Gln Ser Thr Asp Ala Lys
   1 5 10
<210> 255
   <211> 8
   <212> PRT
   <213> Artificial
<220>
   <223> Treponema pallidum membrane protein TMPA
<400> 255
   Lys Ile Glu Gly Asp Leu Lys Lys
   1               5
<210> 256
   <211> 10
   <212> PRT
   <213> Artificial
<220>
   <223> Treponema pallidum membrane protein TMPA
<400> 256
   Glu Leu Arg Pro Gln Leu Glu Glu Ala Asp
   1               5                   10
<210> 257
   <211> 12
   <212> PRT
   <213> Artificial
<220>
   <223> Treponema pallidum membrane protein TMPA
<400> 257
   Asp Ser Gln Tyr Gln Glu Ala Ala Glu Ala Glu Glu
   1               5                   10
<210> 258
   <211> 9
   <212> PRT
   <213> Artificial
<220>
   <223> Treponema pallidum membrane protein TMPA
<400> 258
   Arg Leu Lys Lys Thr Glu Ala Glu Lys
   1               5
<210> 259
   <211> 7
   <212> PRT
   <213> Artificial
<220>
   <223> Treponema pallidum membrane protein TMPA
<400> 259
   Ala Lys Thr Lys Gln Lys Ala
   1 5
<210> 260
   <211> 9
   <212> PRT
   <213> Artificial
<220>
   <223> Treponema pallidum membrane protein TMPA
<400> 260
   Asp Leu Ala Arg Ser Ala Asp Lys Ser
   1 5
<210> 261
   <211> 13
   <212> PRT
   <213> Artificial
<220>
   <223> Treponema pallidum membrane protein TMPA
<400> 261
   Lys Glu Pro Ile Glu Val Glu Pro Leu Pro Asn Asp Arg
   1 5 10
<210> 262
   <211> 15
   <212> PRT
   <213> Artificial
<220>
   <223> Treponema pallidum membrane protein TMPA
<400> 262
   Arg Val Gln Ser Arg Gly Val Glu Asp Gly Gly Arg Ser Pro Lys
   1 5 10 15
<210> 263
   <211> 9
   <212> PRT
   <213> Artificial
<220>
   <223> Islet amyloid polypeptide
<400> 263
   Glu Ser His Gln Val Glu Lys Arg Lys
   1               5
<210> 264
   <211> 8
   <212> PRT
   <213> Artificial
<220>
   <223> Islet amyloid polypeptide
<400> 264
   Asn Pro Gln Met Asp Lys Arg Lys
   1               5
<210> 265
   <211> 8
   <212> PRT
   <213> Artificial
<220>
   <223> Islet amyloid polypeptide
<400> 265
   Asp His Arg Val Asp Lys Arg Lys
   1               5
<210> 266
   <211> 11
   <212> PRT
   <213> Artificial
<220>
   <223> Islet amyloid polypeptide
<400> 266
   Lys Arg Asn Ala Val Glu Val Leu Lys Arg Glu
   1               5                   10
<210> 267
   <211> 11
   <212> PRT
   <213> Artificial
<220>
   <223> Islet amyloid polypeptide
<400> 267
   Lys Arg Asn Val Ala Glu Asp Pro Asn Arg Glu
   1 5 10
<210> 268
   <211> 10
   <212> PRT
   <213> Artificial
<220>
   <223> Islet amyloid polypeptide
<400> 268
   Arg Arg Asn Ala Glu Val Val Asp Val Glu
   1 5 10
<210> 269
   <211> 13
   <212> PRT
   <213> Artificial
<220>
   <223> Collagenase (fibroblast MMP1)
<400> 269
   Leu Lys Asn Asp Gly Arg Gln Val Glu Lys Arg Arg Asn
   1 5 10
<210> 270
   <211> 11
   <212> PRT
   <213> Artificial
<220>
   <223> Collagenase (fibroblast MMP1)
<400> 270
   Lys Asn Asp Gly Arg Gln Val Glu Lys Arg Arg
   1 5 10
<210> 271
   <211> 11
   <212> PRT
   <213> Artificial
<220>
   <223> Collagenase (fibroblast MMP1)
<400> 271
   Lys Asp Asp Trp Arg Lys Ile Pro Lys Gln Arg
   1 5 10
<210> 272
   <211> 7
   <212> PRT
   <213> Artificial
<220>
   <223> Collagenase (fibroblast MMP1)
<400> 272
   Lys Lys Val Glu Arg Gln Arg
   1 5
<210> 273
   <211> 9
   <212> PRT
   <213> Artificial
<220>
   <223> Collagenase (fibroblast MMP1)
<400> 273
   Asp Gly Val Pro Val Glu Lys Lys Arg
   1 5
<210> 274
   <211> 7
   <212> PRT
   <213> Artificial
<220>
   <223> Collagenase (fibroblast MMP1)
<400> 274
   Arg Gly Asp His Arg Asp Asn
   1 5
<210> 275
   <211> 6
   <212> PRT
   <213> Artificial
<220>
   <223> collagenase (fibroblast MMP1)
<400> 275
   Asp Glu His Glu Arg Trp
   1               5
<210> 276
   <211> 9
   <212> PRT
   <213> Artificial
<220>
   <223> collagenase (fibroblast MMP1)
<400> 276
   Asp Glu Asp Asp Arg Trp Thr Lys Asp
   1               5
<210> 277
   <211> 9
   <212> PRT
   <213> Artificial
<220>
   <223> Collagenase (fibroblast MMP1)
<400> 277
   Glu Phe Ala Asp Arg Asp Glu Val Arg
   1               5
<210> 278
   <211> 7
   <212> PRT
   <213> Artificial
<220>
   <223> Collagenase (fibroblast MMP1)
<400> 278
   Glu Val Ala His Arg Asp Glu
   1               5
<210> 279
   <211> 9
   <212> PRT
   <213> Artificial
<220>
   <223> Collagenase (fibroblast MMP1)
<400> 279
   Glu Val Ala Asp Arg Asp Glu Val Arg
   1               5
<210> 280
   <211> 7
   <212> PRT
   <213> Artificial
<220>
   <223> Collagenase (fibroblast MMP1)
<400> 280
   Arg Tyr Asp Glu Tyr Lys Arg
   1               5
<210> 281
   <211> 5
   <212> PRT
   <213> Artificial
<220>
   <223> Schistosoma elastase precursor
<400> 281
   Arg Ser Gly Glu Pro
   1               5
<210> 282
   <211> 10
   <212> PRT
   <213> Artificial
<220>
   <223> Schistosoma elastase precursor
<400> 282
   Arg Asn Gly Asp Gln Gln Gly Ile His His
   1               5                   10
<210> 283
   <211> 6
   <212> PRT
   <213> Artificial
<220>
   <223> Schistosoma elastase precursor
<400> 283
   Ala Arg Gln Arg Arg Pro
   1 5
<210> 284
   <211> 11
   <212> PRT
   <213> Artificial
<220>
   <223> Schistosoma elastase precursor
<400> 284
   Arg Asp Asp Asn Asp Arg Asp Pro Ser Ala Lys
   1 5 10
<210> 285
   <211> 9
   <212> PRT
   <213> Artificial
<220>
   <223> Schistosomin
<400> 285
   Lys Val Ser Lys Arg Pro Asp Tyr Asp
   1 5
<210> 286
   <211> 13
   <212> PRT
   <213> Artificial
<220>
   <223> Schistosomin
<400> 286
   Glu Ala Phe Glu Cys Phe Glu Ser Asp Pro Asn Ala Lys
   1 5 10
<210> 287
   <211> 10
   <212> PRT
   <213> Artificial
<220>
   <223> Schistosomin
<400> 287
   Lys Cys Arg Lys Lys Tyr Glu Phe Cys Arg
   1               5                   10
<210> 288
   <211> 13
   <212> PRT
   <213> Artificial
<220>
   <223> Apolipoprotein(a) human
<400> 288
   His Arg His Gln Arg Thr Pro Glu Asn Tyr Pro Asn Asp
   1               5                   10
<210> 289
   <211> 9
   <212> PRT
   <213> Artificial
<220>
   <223> Apolipoprotein(a) human
<400> 289
   Asp Pro Ser Ile Arg Trp Glu Tyr Cys
   1               5
<210> 290
   <211> 14
   <212> PRT
   <213> Artificial
<220>
   <223> Apolipoprotein(a) rhesus
<400> 290
   His Gln His Lys Arg Thr Pro Glu Asn His Pro Asn Asp Asp
   1               5                   10
<210> 291
   <211> 10
   <212> PRT
   <213> Artificial
<220>
   <223> Apolipoprotein(a) rhesus
<400> 291
   Met Asp Pro Ser Val Arg Arg Glu Tyr Cys
   1 5 10
<210> 292
   <211> 9
   <212> PRT
   <213> Artificial
<220>
   <223> Apolipoprotein(a) rhesus
<400> 292
   Arg Thr Pro Glu Asn Tyr Pro Asn Gly
   1 5
<210> 293
   <211> 8
   <212> PRT
   <213> Artificial
<220>
   <223> Apolipoprotein(a) rhesus
<400> 293
   Leu Glu Ala Pro Ser Lys Gln Ala
   1 5
<210> 294
   <211> 11
   <212> PRT
   <213> Artificial
<220>
   <223> Apolipoprotein(a) rhesus
<400> 294
   Leu Glu Ala Phe Leu Gln Glu Pro Thr Glu Glu
   1 5 10
<210> 295
   <211> 9
   <212> PRT
   <213> Artificial
<220>
   <223> Apolipoprotein(a) rhesus
<400> 295
   Gly His Leu Ala Gly Gly Thr Asp Arg
   1 5
<210> 296
   <211> 11
   <212> PRT
   <213> Artificial
<220>
   <223> Hepatitis delta antigen
<400> 296
   Glu Arg Arg Lys Asp Arg Gly Gly Arg Glu Asp
   1 5 10
<210> 297
   <211> 11
   <212> PRT
   <213> Artificial
<220>
   <223> Hepatitis delta antigen
<400> 297
   Glu Ser Arg Lys Asn Arg Gly Gly Arg Glu Glu
   1 5 10
<210> 298
   <211> 11
   <212> PRT
   <213> Artificial
<220>
   <223> Hepatitis delta antigen
<400> 298
   Glu Ser Lys Gly Lys Arg Ala Gly Arg Glu Gln
   1 5 10
<210> 299
   <211> 13
   <212> PRT
   <213> Artificial
<220>
   <223> REV protein HIV, SIV, VILV, OMWS
<400> 299
   Lys Glu Gly Phe Arg Asn Lys Val Pro Cys Leu Gln Glu
   1               5                   10
<210> 300
   <211> 10
   <212> PRT
   <213> Artificial
<220>
   <223> REV protein HIV, SIV, VILV, OMWS
<400> 300
   Gly Glu Glu Glu Leu Arg Arg Arg Leu Arg
   1               5                   10
<210> 301
   <211> 12
   <212> PRT
   <213> Artificial
<220>
   <223> REV protein HIV, SIV, VILV, OMVVS
<400> 301
   His Glu Arg Glu Glu Glu Leu Arg Lys Arg Leu Arg
   1               5                   10
<210> 302
   <211> 10
   <212> PRT
   <213> Artificial
<220>
   <223> REV protein HIV, SIV, VILV, OMVVS
<400> 302
   Thr Glu Glu Glu Leu Arg Arg Arg Leu Arg
   1               5                   10
<210> 303
   <211> 10
   <212> PRT
   <213> Artificial
<220>
   <223> REV protein HIV, SIV, VILV, OMVVS
<400> 303
   Asn Glu Glu Glu Leu Arg Arg Arg Leu Arg
   1 5 10
<210> 304
   <211> 13
   <212> PRT
   <213> Artificial
<220>
   <223> REV protein HIV, SIV, VILV, OMVVS
<400> 304
   Glu Arg Ala Asp Glu Glu Gly Leu Gln Gly Lys Leu Arg
   1 5 10
<210> 305
   <211> 11
   <212> PRT
   <213> Artificial
<220>
   <223> REV protein HIV, SIV, VILV, OMVVS
<400> 305
   Asp Ser Asp Glu Glu Leu Leu Arg Thr Val Arg
   1 5 10
<210> 306
   <211> 13
   <212> PRT
   <213> Artificial
<220>
   <223> REV protein HIV, SIV, VILV, OMVVS
<400> 306
   Glu Pro Gln Asp Asp Ala Arg Leu Leu Gln Ala Val Lys
   1 5 10
<210> 307
<211> 8
   <212> PRT
   <213> Artificial
<220>
   <223> REV protein HIV, SIV, VILV, OMVVS
<400> 307
   Pro Glu Glu Arg Arg Leu Leu Asx
   1               5
<210> 308
   <211> 10
   <212> PRT
   <213> Artificial
<220>
   <223> REV protein HIV, SIV, VILV, OMVVS
<400> 308
   Trp Arg Asp Met Glu Pro Pro Leu Arg Glu
   1               5                   10
<210> 309
   <211> 10
   <212> PRT
   <213> Artificial
<220>
   <223> REV protein HIV, SIV, VILV, OMWS
<400> 309
   Trp Arg Asp Met Glu Pro Pro Leu Arg Glu
   1               5                   10
<210> 310
   <211> 10
   <212> PRT
   <213> Artificial
<220>
   <223> REV protein HIV, SIV, VILV, OMVVS
<400> 310
   Gln Lys Ser Met Glu Pro Pro Leu Arg Glu
   1               5                   10
<210> 311
   <211> 13
   <212> PRT
   <213> Artificial
<220>
   <223> REV protein HIV, SIV, VILV, OMVVS
<400> 311
   Lys Lys Lys Arg Gly Trp Tyr Lys Trp Leu Arg Lys Leu
   1               5                   10
<210> 312
   <211> 13
   <212> PRT
   <213> Artificial
<220>
   <223> REV protein HIV, SIV, VILV, OMVVS
<400> 312
   Lys Arg Arg Lys Gly Trp Phe Gln Trp Leu Arg Lys Leu
   1               5                   10
<210> 313
   <211> 15
   <212> PRT
   <213> Artificial
<220>
   <223> Angiotensinogen
<400> 313
   Asp Arg Val Tyr Ile His Pro Phe His Leu Leu Tyr His Asn Lys
   1               5                   10                  15
<210> 314
   <211> 15
   <212> PRT
   <213> Artificial
<220>
   <223> Angiotensinogen
<400> 314
   Asp Arg Val Tyr Ile His Pro Phe His Leu Leu Tyr Tyr Ser Lys
   1               5                   10                  15
<210> 315
   <211> 15
   <212> PRT
   <213> Artificial
<220>
   <223> Angiotensinogen
<400> 315
   Asp Arg Val Tyr Ile His Pro Phe His Leu Val Ile His Asn Glu
   1 5 10 15
<210> 316
   <211> 13
   <212> PRT
   <213> Artificial
<220>
   <223> Angiotensinogen
<400> 316
   Glu Asn Pro Ser Val Glu Thr Leu Pro Glu Ser Thr Phe
   1 5 10
<210> 317
   <211> 13
   <212> PRT
   <213> Artificial
<220>
   <223> Angiotensinogen
<400> 317
   Glu Asn Pro Ser Val Glu Thr Leu Pro Glu Pro Thr Phe
   1 5 10
<210> 318
   <211> 13
   <212> PRT
   <213> Artificial
<220>
   <223> Angiotensinogen
<400> 318
   Ala Lys Ala Asn Ala Gly Lys Pro Lys Asp Pro Thr Phe
   1 5 10
<210> 319
   <211> 11
   <212> PRT
   <213> Artificial
<220>
   <223> Angiotensinogen
<400> 319
   Lys Leu Ser Asn Asp Arg Ile Arg Val Gly Glu
   1 5 10
<210> 320
   <211> 11
   <212> PRT
   <213> Artificial
<220>
   <223> Angiotensinogen
<400> 320
   Lys Met Gly Asp Thr Asn Pro Arg Val Gly Glu
   1 5 10
<210> 321
   <211> 11
   <212> PRT
   <213> Artificial
<220>
   <223> Angiotensinogen
<400> 321
   Asn Ile Gly Asp Thr Asn Pro Arg Val Gly Glu
   1 5 10
<210> 322
   <211> 11
   <212> PRT
   <213> Artificial
<220>
   <223> Angiotensinogen
<400> 322
   Glu Leu Glu Ala Asp Glu Arg Glu Pro Thr Glu
   1 5 10
<210> 323
   <211> 7
   <212> PRT
   <213> Artificial
<220>
   <223> Prorenin
<400> 323
   Asp Gly Trp Arg Arg Met Pro
   1 5
<210> 324
   <211> 6
   <212> PRT
   <213> Artificial
<220>
   <223> Prorenin
<400> 324
   Asp Arg Arg Arg Met Pro
   1 5
<210> 325
   <211> 13
   <212> PRT
   <213> Artificial
<220>
   <223> Prorenin
<400> 325
   Lys Arg Ile Phe Leu Lys Arg Met Pro Ser Ile Arg Glu
   1 5 10
<210> 326
   <211> 13
   <212> PRT
   <213> Artificial
<220>
   <223> Prorenin
<400> 326
   Gly Arg Ile Leu Leu Lys Lys Met Pro Ser Val Arg Glu
   1 5 10
<210> 327
   <211> 14
   <212> PRT
   <213> Artificial
<220>
   <223> Prorenin
<400> 327
   Lys Arg Met Pro Ser Ile Arg Glu Ser Leu Lys Glu Arg Gly
   1 5 10
<210> 328
   <211> 14
   <212> PRT
   <213> Artificial
<220>
   <223> Prorenin
<400> 328
   Lys Lys Met Pro Ser Val Arg Glu Ile Leu Glu Glu Arg Gly
   1 5 10
<210> 329
   <211> 8
   <212> PRT
   <213> Artificial
<220>
   <223> Prorenin
<400> 329
   Arg Glu Ile Leu Glu Glu Arg Gly
   1 5
<210> 330
   <211> 12
   <212> PRT
   <213> Artificial
<220>
   <223> Prorenin
<400> 330
   Arg Leu Gly Pro Glu Trp Ser Gln Pro Met Lys Arg
   1 5 10
<210> 331
   <211> 12
   <212> PRT
   <213> Artificial
<220>
   <223> Prorenin
<400> 331
   Arg Ile Ser Ala Glu Trp Gly Glu Phe Ile Lys Lys
   1 5 10
<210> 332
   <211> 12
   <212> PRT
   <213> Artificial
<220>
   <223> Prorenin
<400> 332
   Arg Ile Ser Ala Glu Trp Gly Glu Phe Ile Lys Lys
   1 5 10
<210> 333
   <211> 12
   <212> PRT
   <213> Artificial
<220>
   <223> Prorenin
<400> 333
   Arg Leu Ser Ala Glu Trp Gly Val Phe Thr Lys Arg
   1 5 10
<210> 334
   <211> 14
   <212> PRT
   <213> Artificial
<220>
   <223> Prorenin
<400> 334
   His Lys Leu Phe Asp Ala Ser Asp Ser Ser Ser Tyr Lys His
   1 5 10
<210> 335
   <211> 14
   <212> PRT
   <213> Artificial
<220>
   <223> Prorenin
<400> 335
   His Asn Leu Tyr Asp Ser Ser Glu Ser Ser Ser Tyr Met Glu
   1 5 10
<210> 336
   <211> 14
   <212> PRT
   <213> Artificial
<220>
   <223> Prorenin
<400> 336
   His Ser Leu Tyr Glu Ser Ser Asp Ser Ser Ser Tyr Met Glu
   1 5 10
<210> 337
   <211> 17
   <212> PRT
   <213> Artificial
<220>
   <223> Prorenin
<400> 337
   Lys Glu Asp Val Phe Ser Phe Ser Phe Tyr Tyr Asn Arg Asp Ser Glu
   1 5 10 15
   Asn
<210> 338
   <211> 14
   <212> PRT
   <213> Artificial
<220>
   <223> Prorenin
<400> 338
   Lys Glu Glu Val Phe Ser Val Tyr Tyr Asn Arg Gly Ser His
   1 5 10
<210> 339
   <211> 10
   <212> PRT
   <213> Artificial
<220>
   <223> Prorenin
<400> 339
   Asp Pro Gln His Tyr Glu Gly Asn Phe His
   1 5 10
<210> 340
   <211> 15
   <212> PRT
   <213> Artificial
<220>
   <223> Prorenin
<400> 340
   Glu Lys Leu Met Glu Ala Leu Gly Ala Lys Lys Arg Leu Phe Asp
   1 5 10 15
<210> 341
   <211> 15
   <212> PRT
   <213> Artificial
<220>
   <223> Prorenin
<400> 341
   Gln Leu Ile Met Gln Ala Leu Gly Val Lys Glu Lys Arg Ala Asn
   1 5 10 15
<210> 342
   <211> 15
   <212> PRT
   <213> Artificial
<220>
   <223> Prorenin
<400> 342
   Lys Leu Ile Met Gln Ala Leu Gly Ala Lys Glu Lys Arg Ile Glu
   1 5 10 15
<210> 343
   <211> 12
   <212> PRT
   <213> Artificial
<220>
   <223> Prorenin
<400> 343
   Asp Tyr Val Phe Gln Glu Ser Tyr Ser Ser Lys Lys
   1 5 10
<210> 344
   <211> 12
   <212> PRT
   <213> Artificial
<220>
   <223> Prorenin
<400> 344
   Asp Tyr Val Gln Lys Asn Pro Phe Arg Asn Asp Asp
   1 5 10
<210> 345
   <211> 11
   <212> PRT
   <213> Artificial
<220>
   <223> Prorenin
<400> 345
   Asp Tyr Val Gln Tyr Pro Asn Arg Arg Asp Lys
   1 5 10
<210> 346
   <211> 13
   <212> PRT
   <213> Artificial
<220>
   <223> Prorenin
<400> 346
   Arg Lys Phe Tyr Thr Glu Phe Asp Arg Arg Asn Asn Arg
   1 5 10
<210> 347
   <211> 13
   <212> PRT
   <213> Artificial
<220>
   <223> Prorenin
<400> 347
   Arg Lys Phe Tyr Thr Glu Phe Asp Arg His Asn Asn Arg
   1 5 10
<210> 348
   <211> 13
   <212> PRT
   <213> Artificial
<220>
   <223> Prorenin
<400> 348
   Arg Lys Phe Tyr Thr Glu Phe Asp Arg His Asn Asn Arg
   1 5 10
<210> 349
   <211> 12
   <212> PRT
   <213> Artificial
<220>
   <223> Corticotropin releasing factor binding protein
<400> 349
   Arg Tyr Leu Glu Leu Arg Glu Ala Ala Asp Tyr Asp
   1 5 10
<210> 350
   <211> 12
   <212> PRT
   <213> Artificial
<220>
   <223> Corticotropin releasing factor binding protein
<400> 350
   Arg Tyr Leu Glu Val Gln Glu Ala Ala Val Tyr Asp
   1 5 10
<210> 351
   <211> 12
   <212> PRT
   <213> Artificial
<220>
   <223> Corticotropin releasing factor binding protein
<400> 351
   Lys Arg Asp Val Ala Gly Glu Gln Pro Tyr Arg Arg
   1 5 10
<210> 352
   <211> 12
   <212> PRT
   <213> Artificial
<220>
   <223> Corticotropin releasing factor binding protein
<400> 352
   Lys Arg Asn Leu Ala Glu Glu Gln Pro Tyr Arg Arg
   1 5 10
<210> 353
   <211> 9
   <212> PRT
   <213> Artificial
<220>
   <223> Corticotropin releasing factor binding protein
<400> 353
   Asp His Pro Leu Pro Ser Ala Glu Arg
   1 5
<210> 354
   <211> 9
   <212> PRT
   <213> Artificial
<220>
   <223> Corticotropin releasing factor binding protein
<400> 354
   Asp His Pro Leu Pro Thr Arg Glu Arg
   1 5
<210> 355
   <211> 8
   <212> PRT
   <213> Artificial
<220>
   <223> Corticotropin releasing factor binding protein
<400> 355
   Arg Arg Ser Ile Arg Ser Ser Gln
   1 5
<210> 356
   <211> 8
   <212> PRT
   <213> Artificial
<220>
   <223> Corticotropin releasing factor binding protein
<400> 356
   Arg Arg Ser Val Thr Ser Ser Gln
   1 5
<210> 357
   <211> 10
   <212> PRT
   <213> Artificial
<220>
   <223> Corticotropin releasing factor binding protein
<400> 357
   Asp Pro Ser Lys Met Thr Pro Leu Ala Asp
   1 5 10
<210> 358
   <211> 10
   <212> PRT
   <213> Artificial
<220>
   <223> Corticotropin releasing factor binding protein
<400> 358
   Asp Thr Ser Lys Met Met Leu Leu Val Asp
   1 5 10
<210> 359
   <211> 10
   <212> PRT
   <213> Artificial
<220>
   <223> Corticotropin releasing factor binding protein
<400> 359
   Glu Pro Tyr Glu Leu Glu Asn Pro Asn Gly
   1 5 10
<210> 360
   <211> 10
   <212> PRT
   <213> Artificial
<220>
   <223> Corticotropin releasing factor binding protein
<400> 360
   Glu Pro Leu Glu Leu Glu Thr Ser Thr Arg
   1 5 10

### Sequence listing

### FARNESYLSYNTHETASE

INFORMATION FOR SEQUENCE ID NO 1
   (A) LENGTH:
   (B) TYPE: amino acid
   (D) TOPOLOGY: linear
   -D-V-Y-A-Q-E-K-Q-D-
INFORMATION FOR SEQUENCE ID NO 2
   (A) LENGTH:
   (B) TYPE: amino acid
   (D) TOPOLOGY: linear
   -D-V-H-N-Q-E-K-Q-N-
INFORMATION FOR SEQUENCE ID NO 3
   (A) LENGTH.
   (B) TYPE amino acid
   (D) TOPOLOGY: linear
   -E-I-R-R-E-R-
INFORMATION FOR SEQUENCE ID NO 4
   (A) LENGTH:
   (B) TYPE. amino acid
   (D) TOPOLOGY linear
   -E-D-E-M-G-H-F-E-I-G-D-
INFORMATION FOR SEQUENCE ID NO 5
   (A) LENGTH
   (B) TYPE amino acid
   (D) TOPOLOGY linear
   -E-D-E-L-G-H-P-E-K-G-D-
INFORMATION FOR SEQUENCE ID NO 6
   (A) LENGTH
   (B) TYPE: amino acid
   (D) TOPOLOGY linear
   -A-S-L-L-A-Y-G-M-P-K-E-
INFORMATION FOR SEQUENCE ID NO 7
   (A) LENGTH:
   (B) TYPE: amino acid
   (D) TOPOLOGY: linear
   -E-P-R-K-Q-D-A-D-
INFORMATION FOR SEQUENCE ID NO 8
   (A) LENGTH:
   (B) TYPE: amino acid
   (D) TOPOLOGY: linear
   -E-P-R-K-Q-D-A-E-
INFORMATION FOR SEQUENCE ID NO 9
   (A) LENGTH:
   (B) TYPE: amino acid
   (D) TOPOLOGY: linear
   -N-K-T-V-E-Q-L-G-Q-E-E-
INFORMATION FOR SEQUENCE ID NO 10
   (A) LENGTH:
   (B) TYPE amino acid
   (D) TOPOLOGY linear
   -D-D-I-M-D-S-S-L-T-R-R-
INFORMATION FOR SEQUENCE ID NO 11
   (A) LENGTH:
   (B) TYPE amino acid
   (D) TOPOLOGY linear
   -D-D-I-M-D-S-S-Y-T-R-R-
INFORMATION FOR SEQUENCE ID NO 12
   (A) LENGTH:
   (B) TYPE amino acid
   (D) TOPOLOGY linear
   -D-D-M-M-D-K-S-I-T-R-R-
INFORMATION FOR SEQUENCE ID NO 13
   (A) LENGTH:
   (B) TYPE: amino acid
   (D) TOPOLOGY: linear
   -K-L-Y-C-R-E-Q-P-Y-Y-
INFORMATION FOR SEQUENCE ID NO 14
   (A) LENGTH:
   (B) TYPE: amino acid
   (D) TOPOLOGY: linear
   -K-S-H-F-R-N-E-K-Y-Y-
INFORMATION FOR SEQUENCE ID NO 15
   (A) LENGTH.
   (B) TYPE: amino acid
   (D) TOPOLOGY. linear
   -R-F-T-E-K-R-Y-K-
INFORMATION FOR SEQUENCE ID NO 16
   (A) LENGTH
   (B) TYPE: amino acid
   (D) TOPOLOGY: linear
   -R-Y-T-E-K-R-Y-K-
INFORMATION FOR SEQUENCE ID NO 17
   (A) LENGTH:
   (B) TYPE: amino acid
   (D) TOPOLOGY linear
   -K-F-S-L-K-K-H-S-
INFORMATION FOR SEQUENCE ID NO 18
   (A) LENGTH
   (B) TYPE: amino acid
   (D) TOPOLOGY linear
   -K-V-D-L-S-K-F-S-L-K-K-
INFORMATION FOR SEQUENCE ID NO 19
   (A) LENGTH:
   (B) TYPE: amino acid
   (D) TOPOLOGY: linear
   -K-K-H-S-F-I-V-T-F-K-T-
INFORMATION FOR SEQUENCE ID NO 20
   (A) LENGTH:
   (B) TYPE: amino acid
   (D) TOPOLOGY: linear
   -D-G-E-K-E-H-A-N-A-K-K-
INFORMATION FOR SEQUENCE ID NO 21
   (A) LENGTH
   (B) TYPE: amino acid
   (D) TOPOLOGY: linear
   -D-G-E-K-E-H-A-N-A-L-K-
INFORMATION FOR SEQUENCE ID NO 22
   (A) LENGTH
   (B) TYPE: amino acid
   (D) TOPOLOGY: linear
   -T-D-E-K-D-L-K-Q-A-R-D-
INFORMATION FOR SEQUENCE ID NO 23
   (A) LENGTH:
   (B) TYPE amino acid
   (D) TOPOLOGY: linear
   -K-I-G-T-D-I-Q-D-N-K-
INFORMATION FOR SEQUENCE ID NO 24
   (A) LENGTH
   (B) TYPE amino acid
   (D) TOPOLOGY. linear
   -K-V-G-T-D-I-Q-D-N-K-
INFORMATION FOR SEQUENCE ID NO 25
   (A) LENGTH:
   (B) TYPE: amino acid
   (D) TOPOLOGY: linear
   -Q-R-A-T-P-E-Q-Y-
INFORMATION FOR SEQUENCE ID NO 26
   (A) LENGTH:
   (B) TYPE: amino acid
   (D) TOPOLOGY: linear
   -L-R-A-T-P-Q-Q-R-
INFORMATION FOR SEQUENCE ID NO 27
   (A) LENGTH
   (B) TYPE: amino acid
   (D) TOPOLOGY: linear
   -E-L-A-S-A-E-Q-R-
INFORMATION FOR SEQUENCE ID NO 28
   (A) LENGTH:
   (B) TYPE: amino acid
   (D) TOPOLOGY linear
   - K-E-N-Y-G-Q-K-E-A-E-K-
INFORMATION FOR SEQUENCE ID NO 29
   (A) LENGTH.
   (B) TYPE: amino acid
   (D) TOPOLOGY: linear
   -E-E-N-Y-G-Q-K-D-P-E-K-
INFORMATION FOR SEQUENCE ID NO 30
   (A) LENGTH
   (B) TYPE: amino acid
   (D) TOPOLOGY linear
   -D-E-N-Y-G-K-K-D-S-
INFORMATION FOR SEQUENCE ID NO 31
   (A) LENGTH:
   (B) TYPE: amino acid
   (D) TOPOLOGY: linear
   -R-V-K-A-L-Y-E-E-L-
INFORMATION FOR SEQUENCE ID NO 32
   (A) LENGTH:
   (B) TYPE: amino acid
   (D) TOPOLOGY: linear
   -K-C-K-K-1-F-N-D-L-
INFORMATION FOR SEQUENCE ID NO 33
   (A) LENGTH:
   (B) TYPE: amino acid
   (D) TOPOLOGY: linear
   -K-E-A-E-K-V-A-R-V-K-
INFORMATION FOR SEQUENCE ID NO 34
   (A) LENGTH
   (B) TYPE: amino acid
   (D) TOPOLOGY linear
   -K-D-S-V-A-E-A-K-C-K-
INFORMATION FOR SEQUENCE ID NO 35
   (A) LENGTH
   (B) TYPE: amino acid
   (D) TOPOLOGY linear
   -K-D-P-E-K-V-A-R-
INFORMATION FOR SEQUENCE ID NO 36
   (A) LENGTH
   (B) TYPE: amino acid
   (D) TOPOLOGY linear
   -O-Y-E-E-D-S-Y-S-H-
INFORMATION FOR SEQUENCE ID NO 37
   (A) LENGTH:
   (B) TYPE: amino acid
   (D) TOPOLOGY: linear
   -K-Y-E-E-D-S-Y-N-R-
INFORMATION FOR SEQUENCE ID NO 38
   (A) LENGTH:
   (B) TYPE: amino acid
   (D) TOPOLOGY: linear
   -E-Y-E-E-S-I-A-K-D-
INFORMATION FOR SEQUENCE ID NO 39
   (A) LENGTH:
   (B) TYPE: amino acid
   (D) TOPOLOGY: linear
   -R-K-I-Y-K-R-R-K-
INFORMATION FOR SEQUENCE ID NO 40
   (A) LENGTH.
   (B) TYPE: amino acid
   (D) TOPOLOGY linear
   -N-K-I-Y-K-R-R-K-
INFORMATION FOR SEQUENCE ID NO 41
   (A) LENGTH:
   (B) TYPE: amino acid
   (D) TOPOLOGY: linear
   -N-K-V-Y-K-R-S-K-

### HYDROXY-METHYL-GLUTARYL COENZYME A REDUCTASE

INFORMATION FOR SEQUENCE ID NO 42
   (A) LENGTH
   (B) TYPE amino acid
   (D) TOPOLOGY linear
   -S-Q-D-E-V-R-E-N-
INFORMATION FOR SEQUENCE ID NO 43
   (A) LENGTH:
   (B) TYPE: amino acid
   (D) TOPOLOGY: linear
   -T-Q-N-E-V-V-D-N-
INFORMATION FOR SEQUENCE ID NO 44
   (A) LENGTH:
   (B) TYPE: amino acid
   (D) TOPOLOGY linear
   -E-L-S-R-E-S-R-E-G-R-
INFORMATION FOR SEQUENCE ID NO 45
   (A) LENGTH
   (B) TYPE: amino acid
   (D) TOPOLOGY: linear
   -E-L-S-N-S-N-K-Y-G-R-
INFORMATION FOR SEQUENCE ID NO 46
   (A) LENGTH:
   (B) TYPE: amino acid
   (D) TOPOLOGY linear
   -R-V-L-E-E-E-E-N-K-
INFORMATION FOR SEQUENCE ID NO 47
   (A) LENGTH
   (B) TYPE: amino acid
   (D) TOPOLOGY: linear
   -E-V-L-E-E-E-E-D-R-K-
INFORMATION FOR SEQUENCE ID NO 48
   (A) LENGTH.
   (B) TYPE amino acid
   (D) TOPOLOGY linear
   -K-G-V-S-S-S-T-R-K-
INFORMATION FOR SEQUENCE ID NO 49
   (A) LENGTH:
   (B) TYPE: amino acid
   (D) TOPOLOGY: linear
   -S-V-L-E-E-E-E-D-N-K-
INFORMATION FOR SEQUENCE ID NO 50
   (A) LENGTH:
   (B) TYPE: amino add
   (D) TOPOLOGY: linear
   -D-E-N-V-S-K-R-I-E-P-
INFORMATION FOR SEQUENCE ID NO 51
   (A) LENGTH:
   (B) TYPE: amino acici
   (D) TOPOLOGY: linear
   -D-D-M-M-P-K-R-V-E-
INFORMATION FOR SEQUENCE ID NO 52
   (A) LENGTH.
   (B) TYPE amino acid
   (D) TOPOLOGY: linear
   -D-P-T-M-P-L-W-E-F-
INFORMATION FOR SEQUENCE ID NO 53
   (A) LENGTH
   (B) TYPE amino acid
   (D) TOPOLOGY linear
   -K-K-V-P-D-N-C-C-R-R-E-
INFORMATION FOR SEQUENCE ID NO 54
   (A) LENGTH
   (B) TYPE. amino acid
   (D) TOPOLOGY linear
   -K-K-A-P-D-N-C-C-R-R-E-
INFORMATION FOR SEQUENCE ID NO 55
   (A) LENGTH:
   (B) TYPE: amino acid
   (D) TOPOLOGY: linear
   -K-K-Q-I-E-S-C-C-R-R-E-
INFORMATION FOR SEQUENCE ID NO 56
   (A) LENGTH:
   (B) TYPE: amino acid
   (D) TOPOLOGY: linear
   -Q-K-C-D-S-V-E-E-
INFORMATION FOR SEQUENCE ID NO 57
   (A) LENGTH:
   (B) TYPE: amino acid
   (D) TOPOLOGY: linear
   -E-K-L-S-S-V-E-E-
INFORMATION FOR SEQUENCE ID NO 58
   (A) LENGTH
   (B) TYPE: amino acid
   (D) TOPOLOGY linear
   -H-V-S-T-T-E-E-
INFORMATION FOR SEQUENCE ID NO 59
   (A) LENGTH.
   (B) TYPE: amino acid
   (D) TOPOLOGY linear
   -E-T-L-I-S-D-Q-
INFORMATION FOR SEQUENCE ID NO 60
   (A) LENGTH:
   (B) TYPE. amino acid
   (D) TOPOLOGY: linear
   -E-E-T-G-I-N-R-E-R-K-V-E-
INFORMATION FOR SEQUENCE ID NO 61 (A) LENGTH:
   (B) TYPE: amino acid
   (D) TOPOLOGY: linear
   -E-E-P-G-V-S-Q-D-R-K-V-E-
INFORMATION FOR SEQUENCE ID NO 62
   (A) LENGTH:
   (B) TYPE: amino acid
   (D) TOPOLOGY: linear
   -E-A-S-S-K-E-E-T-E-A-
INFORMATION FOR SEQUENCE ID NO 63
   (A) LENGTH:
   (B) TYPE: amino acid
   (D) TOPOLOGY. linear
   -T-D-F-V-T-A-S-P-R-N-
INFORMATION FOR SEQUENCE ID NO 64
   (A) LENGTH
   (B) TYPE: amino acid
   (D) TOPOLOGY linear
   -E-P-E-I-E-L-P-R-E-P-R-P-N-E-E-
INFORMATION FOR SEQUENCE ID NO 65
   (A) LENGTH.
   (B) TYPE: amino acid
   (D) TOPOLOGY: linear
   -E-L-E-I-E-L-P-S-E-P-R-P-N-E-E-
INFORMATION FOR SEQUENCE ID NO 66
   (A) LENGTH
   (B) TYPE: amino acid
   (D) TOPOLOGY linear
   -N-T-M-F-D-L-P-E-E-P-R-P-L-D-E-
INFORMATION FOR SEQUENCE ID NO 67
   (A) LENGTH:
   (B) TYPE: amino acid
   (D) TOPOLOGY: linear
   -E-V-E-W-V-L-E-T-E-L-K-A-P-R-P-
INFORMATION FOR SEQUENCE ID NO 68
   (A) LENGTH:
   (B) TYPE: amino acid
   (D) TOPOLOGY: linear
   -R-E-P-R-P-N-E-E-C-
INFORMATION FOR SEQUENCE ID NO 69
   (A) LENGTH
   (B) TYPE: amino acid
   (D) TOPOLOGY: linear
   -E-L-K-A-P-R-P-M-
INFORMATION FOR SEQUENCE ID NO 70
   (A) LENGTH
   (B) TYPE: amino acid
   (D) TOPOLOGY: linear
   -E-E-P-R-P-L-D-E-C-
INFORMATION FOR SEQUENCE ID NO 71
   (A) LENGTH:
   (B) TYPE. amino acid
   (D) TOPOLOGY: linear
   -H-E-R-G-U-S-I-R-R-Q-L-L-S-K-K-
INFORMATION FOR SEQUENCE ID NO 72
   (A) LENGTH
   (B) TYPE: amino acid
   (D) TOPOLOGY linear
   -P-R-E-G-V-A-I-R-R-O-M-L-S-D-K-
INFORMATION FOR SEQUENCE ID NO 73
   (A) LENGTH:
   (B) TYPE: amino acid
   (D) TOPOLOGY: linear
   -P-E-R-G-V-A-V-R-R-Q-I-I-S-K-
INFORMATION FOR SEQUENCE ID NO 74
   (A) LENGTH:
   (B) TYPE: amino acid
   (D) TOPOLOGY: linear
   -P-F-R-A-V-E-L-R-R-L-D-L-
INFORMATION FOR SEQUENCE ID NO 75
   (A) LENGTH.
   (B) TYPE: amino acid
   (D) TOPOLOGY: linear
   -L-Q-Y-L-P-Y-R-D-Y-N-
INFORMATION FOR SEQUENCE ID NO 76
   (A) LENGTH:
   (B) TYPE amino acid
   (D) TOPOLOGY. linear
   -I-E-R-I-P-Y-K-D-Y-D-
INFORMATION FOR SEQUENCE ID NO 77
   (A) LENGTH:
   (B) TYPE: amino acid
   (D) TOPOLOGY: linear
   -L-Q-S-L-P-Y-K-N-Y-N-
INFORMATION FOR SEQUENCE ID NO 78
   (A) LENGTH:
   (B) TYPE amino acid
   (D) TOPOLOGY: linear
   -L-E-K-L-P-Y-A-S-Y-D-
INFORMATION FOR SEQUENCE ID NO 79
   (A) LENGTH:
   (B) TYPE: amino acid
   (D) TOPOLOGY: linear
   -C-L-D-E-K-E -F-Q-
INFORMATION FOR SEQUENCE ID NO 80
   (A) LENGTH:
   (B) TYPE: amino acid
   (D) TOPOLOGY: linear
   -C-L-D-G-K-E-Y-Q-
INFORMATION FOR SEQUENCE ID NO 81
   (A) LENGTH:
   (B) TYPE: amino acid
   (D) TOPOLOGY: linear
   -L-L-N-N-K-E-Y-Q-
INFORMATION FOR SEQUENCE ID NO 82
   (A) LENGTH:
   (B) TYPE: amino acid
   (D) TOPOLOGY: linear
   -L-L-D-G-O-E-F-Q-
INFORMATION FOR SEQUENCE ID NO 83
   (A) LENGTH:
   (B) TYPE: amino acid
   (D) TOPOLOGY: linear
   -L-L-D-G-R-S-H-Y-
INFORMATION FOR SEQUENCE ID NO 84
   (A) LENGTH:
   (B) TYPE: amino acid
   (D) TOPOLOGY linear
   -R-L-P-R-A-C-D-S-A-E-V-K-
INFORMATION FOR SEQUENCE ID NO 85
   (A) LENGTH:
   (B) TYPE: amino acid
   (D) TOPOLOGY: linear
   -R-L-P-T-A-C-D-A-A-E-V-K-
INFORMATION FOR SEQUENCE ID NO 86
   (A) LENGTH:
   (B) TYPE: amino acid
   (D) TOPOLOGY: linear
   -R-L-P-S-A-Q-E-A-G-A-I-K-
INFORMATION FOR SEQUENCE ID NO 87
   (A) LENGTH:
   (B) TYPE: amino acid
   (D) TOPOLOGY: linear
   -E-K-A-L-S-K-L-H-E-Y-
INFORMATION FOR SEQUENCE ID NO 88
   (A) LENGTH.
   (B) TYPE: amino acid
   (D) TOPOLOGY. linear
   -E-K-A-L-V-K-L-Q-E-F-
INFORMATION FOR SEQUENCE ID NO 89
   (A) LENGTH:
   (B) TYPE: amino acid
   (D) TOPOLOGY: linear
   -E-Q-A-L-A-R-L-Q-E-E-
INFORMATION FOR SEQUENCE ID NO 90
   (A) LENGTH.
   (B) TYPE: amino acid
   (D) TOPOLOGY: linear
   -E-Q-A-L-H-A-L-Q-T-M-
INFORMATION FOR SEQUENCE ID NO 91
   (A) LENGTH:
   (B) TYPE: amino acid
   (D) TOPOLOGY: linear
   -C-K-D-N-P-G-E-N-A-R-
INFORMATION FOR SEQUENCE ID NO 92
   (A) LENGTH:
   (B) TYPE: amino acid
   (D) TOPOLOGY: linear
   -S-T-E-T-P-G-K-N-A-C-
INFORMATION FOR SEQUENCE ID NO 93
   (A) LENGTH:
   (B) TYPE: amino acid
   (D) TOPOLOGY: linear
   -N-I-H-G-S-G-L-N-A-S-
INFORMATION FOR SEQUENCE ID NO 94
   (A) LENGTH.
   (B) TYPE: amino acid
   (D) TOPOLOGY: linear
   -H-N-R-S-K-I-N-L-Q-D-
INFORMATION FOR SEQUENCE ID NO 95
   (A) LENGTH.
   (B) TYPE: amino acid
   (D) TOPOLOGY: linear
   -H-N-R-S-A-L-N-I-D-E-
GLUCAGON PRECURSOR INFORMATION FOR SEQUENCE ID NO 96
   (A) LENGTH:
   (B) TYPE: amino acid
   (D) TOPOLOGY linear
   -D-Y-S-K-Y-L-D-S-
INFORMATION FOR SEQUENCE ID NO 97
   (A) LENGTH:
   (B) TYPE: amino acid
   (D) TOPOLOGY: linear
   -D-S-R-R-A-Q-D-
INFORMATION FOR SEQUENCE ID NO 98
   (A) LENGTH:
   (B) TYPE: amino acid
   (D) TOPOLOGY: linear
   -D-Y-S-K-Y-L-D-S-R-R-A-Q-D-
INFORMATION FOR SEQUENCE ID NO 99
   (A) LENGTH
   (B) TYPE: amino acid
   (D) TOPOLOGY: linear
   -D-V-A-S-L-T-D-Y-L-K-S-K-R-
INFORMATION FOR SEQUENCE ID NO 100
   (A) LENGTH.
   (B) TYPE. amino acid
   (D) TOPOLOGY linear
   -D-Y-S-K-Y-M-D-N-R-R-A-K-D-
INFORMATION FOR SEQUENCE ID NO 101
   (A) LENGTH:
   (B) TYPE: amino acid
   (D) TOPOLOGY: linear
   -D-Y-S-K-Y-L-D-N-R-R-A-K-D-
INFORMATION FOR SEQUENCE ID NO 102
   (A) LENGTH.
   (B) TYPE. amino acid
   (D) TOPOLOGY linear
   -D-Y-S-K-Y-L-D-S-R-R-A-Q-Q-
INFORMATION FOR SEQUENCE ID NO 103
   (A) LENGTH:
   (B) TYPE: amino acid
   (D) TOPOLOGY: linear
   -D-Y-S-K-Y-L-D-S-
INFORMATION FOR SEQUENCE ID NO 104
   (A) LENGTH:
   (B) TYPE: amino acid
   (D) TOPOLOGY: linear
   -D-S-R-R-A-Q-D-
INFORMATION FOR SEQUENCE ID NO 105
   (A) LENGTH
   (B) TYPE: amino acid
   (D) TOPOLOGY: linear
   -D-Y-S-K-Y-L-D-S-R-R-A-Q-D-
INFORMATION FOR SEQUENCE ID NO 106
   (A) LENGTH.
   (B) TYPE amino acid
   (D) TOPOLOGY linear
   -D-V-A-S-L-T-D-Y-L-K-S-K-R-
INFORMATION FOR SEQUENCE ID NO 107
   (A) LENGTH:
   (B) TYPE: amino acid
   (D) TOPOLOGY linear
   -D-Y-S-K-Y-M-D-N-R-R-A-K-D-
INFORMATION FOR SEQUENCE ID NO 108
   (A) LENGTH
   (B) TYPE amino acid
   (D) TOPOLOGY linear
   -D-Y-S-K-Y-L-D-N-R-R-A-K-D-
INFORMATION FOR SEQUENCE ID NO 109
   (A) LENGTH:
   (B) TYPE: amino acid
   (D) TOPOLOGY: linear
   -D-Y-S-K-Y-L-D-S-R-R-A-Q-Q-
INFORMATION FOR SEQUENCE ID NO 110
   (A) LENGTH:
   (B) TYPE: amino acid
   (D) TOPOLOGY: linear
   -D-Y-S-K-F-L-D-T-R-R-A-Q-D-
INFORMATION FOR SEQUENCE ID NO 111
   (A) LENGTH.
   (B) TYPE: amino acid
   (D) TOPOLOGY: linear
   -D-Y-S-K-Y-Q-E-E-R-M-A-Q-D-
INFORMATION FOR SEQUENCE ID NO 112
   (A) LENGTH.
   (B) TYPE amino acid
   (D) TOPOLOGY: linear
   -D-Y-S-K-Y-L-E-T-R-R-A-Q-D-
INFORMATION FOR SEQUENCE ID NO 113
   (A) LENGTH:
   (B) TYPE: amino acid
   (D) TOPOLOGY. linear
   -D-Y-S-K-Y-L-D-N-R-R-T-K-D-
INFORMATION FOR SEQUENCE ID NO 114
   (A) LENGTH:
   (B) TYPE: amino acid
   (D) TOPOLOGY. linear
   -D-M-S-S-Y-L-E-E-K-A-A-K-E-
INFORMATION FOR SEQUENCE ID NO 115
   (A) LENGTH:
   (B) TYPE: amino acid
   (D) TOPOLOGY: linear
   -D-F-N-K-A-L-D-I-K-A-A-Q-E-
INFORMATION FOR SEQUENCE ID NO 116
   (A) LENGTH:
   (B) TYPE: amino add
   (D) TOPOLOGY: linear
   -D-P-L-S-D-P-D-Q-M-
INFORMATION FOR SEQUENCE ID NO 117
   (A) LENGTH:
   (B) TYPE: amino acid
   (D) TOPOLOGY: linear
   -N-E-D-K-R-H-S-Q-
INFORMATION FOR SEQUENCE ID NO 118
   (A) LENGTH.
   (B) TYPE: amino acid
   (D) TOPOLOGY: linear
   -K-D-E-P-R-E-L-S-N-M-K-R-H-S-E-
INFORMATION FOR SEQUENCE ID NO 119
   (A) LENGTH.
   (B) TYPE. amino acid
   (D) TOPOLOGY: linear
   -R-E-L-S-N-M-K-R-H-
INFORMATION FOR SEQUENCE ID NO 120
   (A) LENGTH
   (B) TYPE. amino acid
   (D) TOPOLOGY linear
   -E-A-R-E-L-S-T-P-K-X-H-S-E-
INFORMATION FOR SEQUENCE ID NO 121
   (A) LENGTH:
   (B) TYPE: amino acid
   (D) TOPOLOGY: linear
   -E-D-P-D-Q-I-N-E-D-K-R-H-
INFORMATION FOR SEQUENCE ID NO 122
   (A) LENGTH:
   (B) TYPE: amino acid
   (D) TOPOLOGY: linear
   -D-D-P-D-Q-M-N-E-D-K-R-H-S-Q-
INFORMATION FOR SEQUENCE ID NO 123
   (A) LENGTH:
   (B) TYPE: amino acid
   (D) TOPOLOGY: linear
   -G-D-P-D-Q-I-N-E-D-K-R-H-S-Q-
INFORMATION FOR SEQUENCE ID NO 124
   (A) LENGTH:
   (B) TYPE: amino acid
   (D) TOPOLOGY linear
   -D-E-S-R-Q-L-N-E-V-K-R-H-S-Q-
INFORMATION FOR SEQUENCE ID NO 125
   (A) LENGTH:
   (B) TYPE: amino acid
   (D) TOPOLOGY: linear
   -M-N-T-K-R-N-R-N-N-
INFORMATION FOR SEQUENCE ID NO 126
   (A) LENGTH.
   (B) TYPE: amino acid
   (D) TOPOLOGY: linear
   -K-R-H-D-E-F-E-R-H-
INFORMATION FOR SEQUENCE ID NO 127
   (A) LENGTH:
   (B) TYPE: amino acid
   (D) TOPOLOGY: linear
   -M-N-T-K-R-N-R-N-R-N-N-K-R-H-D-E-F-E-R-H-
INFORMATION FOR SEQUENCE ID NO 128
   (A) LENGTH:
   (B) TYPE: amino acid
   (D) TOPOLOGY: linear
   -N-K-R-S-G-V-A-E-K-R-
INFORMATION FOR SEQUENCE ID NO 129
   (A) LENGTH:
   (B) TYPE: amino acid
   (D) TOPOLOGY linear
   -V-K-R-N-R-N-N-I-A-K-R-
INFORMATION FOR SEQUENCE ID NO 130
   (A) LENGTH:
   (B) TYPE: amino acid
   (D) TOPOLOGY. linear
   -K-N-T-K-R-N-R-N-E-
INFORMATION FOR SEQUENCE ID NO 131
   (A) LENGTH:
   (B) TYPE: amino acid
   (D) TOPOLOGY: linear
   -T-K-R-N-G-O-O-G-Q-E-D-K-
INFORMATION FOR SEQUENCE ID NO 132
   (A) LENGTH:
   (B) TYPE. amino acid
   (D) TOPOLOGY linear
   -Q-E-D-K-E-N-D-K-F-
INFORMATION FOR SEQUENCE ID NO 133
   (A) LENGTH:
   (B) TYPE: amino acid
   (D) TOPOLOGY: linear
   -K-R-H-S-E-F-E-R-H-A-E-
INFORMATION FOR SEQUENCE ID NO 134
   (A) LENGTH:
   (B) TYPE: amino acid
   (D) TOPOLOGY: linear
   -Q-D-T-E-E-K-S-R-
INFORMATION FOR SEQUENCE ID NO 135
   (A) LENGTH:
   (B) TYPE: amino acid
   (D) TOPOLOGY linear
   -Q-D-T-E-E-K-P-R-
INFORMATION FOR SEQUENCE ID NO 136
   (A) LENGTH:
   (B) TYPE: amino acid
   (D) TOPOLOGY linear
   -Q-D-T-E-E-N-A-R-
INFORMATION FOR SEQUENCE ID NO 137
   (A) LENGTH:
   (B) TYPE: amino acid
   (D) TOPOLOGY linear
   -D-Q-D-P-D-R-N-S-M-
INFORMATION FOR SEQUENCE ID NO 138
   (A) LENGTH.
   (B) TYPE: amino acid
   (D) TOPOLOGY linear
   -K-G-R-G-R-R-D-F-P-E-E-
INFORMATION FOR SEQUENCE ID NO 139
   (A) LENGTH:
   (B) TYPE: amino acid
   (D) TOPOLOGY: linear
   -D-R-L-K-A-Q-V-R-R-E-
INFORMATION FOR SEQUENCE ID NO 140
   (A) LENGTH:
   (B) TYPE: amino acid
   (D) TOPOLOGY: linear
   -A-K-L-K-S-G-K-V-
INFORMATION FOR SEQUENCE ID NO 141
   (A) LENGTH:
   (B) TYPE: amino acid
   (D) TOPOLOGY: linear
   -K-S-G-Q-P-K-P-E-
INFORMATION FOR SEQUENCE ID NO 142
   (A) LENGTH:
   (B) TYPE: amino acid
   (D) TOPOLOGY: linear
   -K-Q-G-Q-D-R-R-E-

### GASTRIN PRECURSOR

INFORMATION FOR SEQUENCE ID NO 143
   (A) LENGTH
   (B) TYPE: amino acid
   (D) TOPOLOGY: linear
   -A-D-P-S-K-K-Q-
INFORMATION FOR SEQUENCE ID NO 144
   (A) LENGTH:
   (B) TYPE. amino acid
   (D) TOPOLOGY. linear
   -R-T-D-L-S-K-K-Q-
INFORMATION FOR SEQUENCE ID NO 145
   (A) LENGTH:
   (B) TYPE: amino acid
   (D) TOPOLOGY: linear
   -A-D-L-S-K-K-Q-
INFORMATION FOR SEQUENCE ID NO 146
   (A) LENGTH:
   (B) TYPE: amino acid
   (D) TOPOLOGY: linear
   -D-L-S-K-K-Q-R-
INFORMATION FOR SEQUENCE ID NO 147
   (A) LENGTH:
   (B) TYPE: amino acid
   (D) TOPOLOGY: linear
   -W-L-E-E-E-E-E-A-
INFORMATION FOR SEQUENCE ID NO 148
   (A) LENGTH
   (B) TYPE: amino acid
   (D) TOPOLOGY linear
   -W-M-E-E-E-E-A-
INFORMATION FOR SEQUENCE ID NO 149
   (A) LENGTH:
   (B) TYPE: amino acid
   (D) TOPOLOGY linear
   -W-V-E-E-E-E-A-
INFORMATION FOR SEQUENCE ID NO 150
   (A) LENGTH:
   (B) TYPE: amino acid
   (D) TOPOLOGY linear
   -W-A-E-E-E-E-A-
INFORMATION FOR SEQUENCE ID NO 151
   (A) LENGTH:
   (B) TYPE: amino acid
   (D) TOPOLOGY: linear
   -P-M-E-E-E-E-E-A-
INFORMATION FOR SEQUENCE ID NO 152
   (A) LENGTH:
   (B) TYPE: amino acid
   (D) TOPOLOGY: linear
   -D-F-G-R-R-S-A-E-D-E-N-
INFORMATION FOR SEQUENCE ID NO 153
   (A) LENGTH
   (B) TYPE: amino acid
   (D) TOPOLOGY: linear
   -M-D-F-G-R-R-S-
INFORMATION FOR SEQUENCE ID NO 154
   (A) LENGTH
   (B) TYPE amino acid
   (D) TOPOLOGY: linear
   -R-R-S-A-E-D-E-
INFORMATION FOR SEQUENCE ID NO 155
   (A) LENGTH:
   (B) TYPE: amino acid
   (D) TOPOLOGY: linear
   -R-R-S-A-E-D-G-D-Q-H-P-
INFORMATION FOR SEQUENCE ID NO 156
   (A) LENGTH
   (B) TYPE: amino acid
   (D) TOPOLOGY: linear
   - R-D-L-E-L-P-W-L-E-
INFORMATION FOR SEQUENCE ID NO 157
   (A) LENGTH:
   (B) TYPE: amino acid
   (D) TOPOLOGY: linear
   -R-G-K-E-P-H-E-L-D-R-
INFORMATION FOR SEQUENCE ID NO 158
   (A) LENGTH:
   (B) TYPE: amino acid
   (D) TOPOLOGY: linear
   -R-G-Q-E-P-L-R-
INFORMATION FOR SEQUENCE ID NO 159
   (A) LENGTH:
   (B) TYPE: amino acid
   (D) TOPOLOGY: linear
   -E-P-H-W-L-N-R-
INFORMATION FOR SEQUENCE ID NO 160
   (A) LENGTH:
   (B) TYPE: amino acid
   (D) TOPOLOGY: linear
   -K-P-R-S-Q-Q-P-D-
INFORMATION FOR SEQUENCE ID NO 161
   (A) LENGTH:
   (B) TYPE: amino acid
   (D) TOPOLOGY: linear
   -K-P-G-F-Q-L-Q-D-
INFORMATION FOR SEQUENCE ID NO 162
   (A) LENGTH:
   (B) TYPE: amino acid
   (D) TOPOLOGY: linear
   -K-P-H-S-H-L-Q-D-50
INFORMATION FOR SEQUENCE ID NO 163
   (A) LENGTH:
   (B) TYPE: amino acid
   (D) TOPOLOGY: linear
   -K-P-R-S-Q-L-Q-D-

### GONADOLIBERIN PRECURSOR

INFORMATION FOR SEQUENCE ID NO 164
   (A) LENGTH:
   (B) TYPE: amino acid
   (D) TOPOLOGY: linear
   -Q-H-W-S-Y-G-L-R-P-G-G-K-
INFORMATION FOR SEQUENCE ID NO 165
   (A) LENGTH
   (B) TYPE: amino acid
   (D) TOPOLOGY: linear
   -G-L-Q-P-G- G-K-R-D-
INFORMATION FOR SEQUENCE ID NO 166
   (A) LENGTH:
   (B) TYPE: amino acid
   (D) TOPOLOGY: linear
   -H-G-W-L-P-G-G-K-R-D-
INFORMATION FOR SEQUENCE ID NO 167
   (A) LENGTH:
   (B) TYPE amino acid
   (D) TOPOLOGY. linear
   -G-W-L-P-G-G-K-R-D-
INFORMATION FOR SEQUENCE ID NO 168
   (A) LENGTH
   (B) TYPE amino acid
   (D) TOPOLOGY linear
   -H-G-W-Y-P-G-G-K-R-D-
INFORMATION FOR SEQUENCE ID NO 169
   (A) LENGTH:
   (B) TYPE: amino acid
   (D) TOPOLOGY: linear
   -Q-H-Y-S-L-E-W-K-P-G-
INFORMATION FOR SEQUENCE ID NO 170
   (A) LENGTH:
   (B) TYPE: amino acid
   (D) TOPOLOGY: linear
   -R-P-G-G-K-R-D-A-E-
INFORMATION FOR SEQUENCE ID NO 171
   (A) LENGTH:
   (B) TYPE: amino acid
   (D) TOPOLOGY: linear
   -R-P-G-G-K-R-N-T-E-H-
INFORMATION FOR SEQUENCE ID NO 172
   (A) LENGTH
   (B) TYPE: amino acid
   (D) TOPOLOGY: linear
   -Q-P-G-G-K-R-D-A-E-

### PLASMINOGEN ACTIVATOR INHIBITOR 1

INFORMATION FOR SEQUENCE ID NO 173
   (A) LENGTH:
   (B) TYPE: amino acid
   (D) TOPOLOGY: linear
   -Q-A-S-K-D-R-
INFORMATION FOR SEQUENCE ID NO 174
   (A) LENGTH:
   (B) TYPE: amino acid
   (D) TOPOLOGY. linear
   -K-I-D-D-K-G-
INFORMATION FOR SEQUENCE ID NO 175
   (A) LENGTH:
   (B) TYPE: amino acid
   (D) TOPOLOGY: linear
   -W-N-K-D-E-
INFORMATION FOR SEQUENCE ID NO 176
   (A) LENGTH:
   (B) TYPE: amino acid
   (D) TOPOLOGY: linear
   -E-V-E-R-A-R-
INFORMATION FOR SEQUENCE ID NO 177
   (A) LENGTH:
   (B) TYPE: amino acid
   (D) TOPOLOGY: linear
   -H-R-R-L-F-H-K-S-D-
INFORMATION FOR SEQUENCE ID NO 178
   (A) LENGTH:
   (B) TYPE amino acid
   (D) TOPOLOGY: linear
   -K-T-P-F-P-D-S-
INFORMATION FOR SEQUENCE ID NO 179
   (A) LENGTH
   (B) TYPE: amino acid
   (D) TOPOLOGY: linear
   -Y-E-K-E-V-P-
INFORMATION FOR SEQUENCE ID NO 180
   (A) LENGTH:
   (B) TYPE. amino acid
   (D) TOPOLOGY linear
   -E-V-D-L-R-K-P-
INFORMATION FOR SEQUENCE ID NO 181
   (A) LENGTH:
   (B) TYPE: amino acid
   (D) TOPOLOGY: linear
   -K-V-K-I-E- V-N-E-
INFORMATION FOR SEQUENCE ID NO 182
   (A) LENGTH:
   (B) TYPE: amino acid
   (D) TOPOLOGY: linear
   -A-R-M-A-P-E-E-
INFORMATION FOR SEQUENCE ID NO 183
   (A) LENGTH:
   (B) TYPE: amino acid
   (D) TOPOLOGY: linear
   -R-A-S-K-D-R-
INFORMATION FOR SEQUENCE ID NO 184
   (A) LENGTH:
   (B) TYPE: amino acid
   (D) TOPOLOGY: linear
   -K-I-E-E-K-G-
INFORMATION FOR SEQUENCE ID NO 185
   (A) LENGTH:
   (B) TYPE: amino acid
   (D) TOPOLOGY: linear
   -K-V-N-E-K-G-
INFORMATION FOR SEQUENCE ID NO 186
   (A) LENGTH
   (B) TYPE. amino acid
   (D) TOPOLOGY linear
   -N-I-S-E-R-G-
INFORMATION FOR SEQUENCE ID NO 187
   (A) LENGTH.
   (B) TYPE: amino acid
   (D) TOPOLOGY: linear
   -W-N-K-N-E-
INFORMATION FOR SEQUENCE ID NO 188
   (A) LENGTH:
   (B) TYPE: amino acid
   (D) TOPOLOGY: linear
   -K-M-P-F-P-E-S-
INFORMATION FOR SEQUENCE ID NO 189
   (A) LENGTH:
   (B) TYPE: amino acid
   (D) TOPOLOGY: linear
   -K-T-P-F-L-E-A-
INFORMATION FOR SEQUENCE ID NO 190
   (A) LENGTH:
   (B) TYPE: amino acid
   (D) TOPOLOGY: linear
   -H-H-R-L-F-H-K-S-D-
INFORMATION FOR SEQUENCE ID NO 191
   (A) LENGTH:
   (B) TYPE: amino acid
   (D) TOPOLOGY linear
   -H-Q-R-L-F-H-K-S-D-
INFORMATION FOR SEQUENCE ID NO 192
   (A) LENGTH
   (B) TYPE: amino acid
   (D) TOPOLOGY: linear
   -E-V-D-L-R-G-P-L-E-K-
INFORMATION FOR SEQUENCE ID NO 193
   (A) LENGTH:
   (B) TYPE: amino acid
   (D) TOPOLOGY: linear
   -E-1-D-L-R-R-P-L-E-
INFORMATION FOR SEQUENCE ID NO 194
   (A) LENGTH:
   (B) TYPE: amino add
   (D) TOPOLOGY: linear
   -A-R-M-A-P-T-E-M-

### PLASMINOGEN ACTIVATOR INHIBITOR 2

INFORMATION FOR SEQUENCE ID NO 195
   (A) LENGTH:
   (B) TYPE: amino acid
   (D) TOPOLOGY: linear
   -K-W-K-T-P-F-E-K-
INFORMATION FOR SEQUENCE ID NO 196
   (A) LENGTH
   (B) TYPE. amino acid
   (D) TOPOLOGY linear
   -R-E-K-L-N-I-G-Y-I-E-D-L-K-
INFORMATION FOR SEQUENCE ID NO 197
   (A) LENGTH:
   (B) TYPE: amino acid
   (D) TOPOLOGY: linear
   -H-A-K-L-N-I-G-Y-I-K-D-L-K-
INFORMATION FOR SEQUENCE ID NO 198
   (A) LENGTH
   (B) TYPE: amino acid
   (D) TOPOLOGY: linear
   -K-D-K-M-A-E-D-E-V-E-
INFORMATION FOR SEQUENCE ID NO 199
   (A) LENGTH:
   (B) TYPE: amino acid
   (D) TOPOLOGY: linear
   -K-D-T-L-D-E-D-D-
INFORMATION FOR SEQUENCE ID NO 200
   (A) LENGTH:
   (B) TYPE: amino acid
   (D) TOPOLOGY: linear
   -K-L-E-E-H-Y-E-L-R-
INFORMATION FOR SEQUENCE ID NO 201
   (A) LENGTH:
   (B) TYPE: amino acid
   (D) TOPOLOGY linear
   -K-L-A-Q-S-Y-E-L-K-
INFORMATION FOR SEQUENCE ID NO 202
   (A) LENGTH:
   (B) TYPE amino acid
   (D) TOPOLOGY: linear
   -E-R-N-D-L-F-L-S-E-
INFORMATION FOR SEQUENCE ID NO 203
   (A) LENGTH
   (B) TYPE amino acid
   (D) TOPOLOGY: linear
   -D-V-N-E-E-G-T-E-
INFORMATION FOR SEQUENCE ID NO 204
   (A) LENGTH
   (B) TYPE: amino acid
   (D) TOPOLOGY: linear
   -D-V-T-E-E-G-T-V-
INFORMATION FOR SEQUENCE ID NO 205
   (A) LENGTH:
   (B) TYPE: amino acid
   (D) TOPOLOGY: linear
   -R-G-S-T-E-D-E-Q-
INFORMATION FOR SEQUENCE ID NO 206
   (A) LENGTH:
   (B) TYPE: amino acid
   (D) TOPOLOGY: linear
   -G-G-N-T-E-Q-Q-
INFORMATION FOR SEQUENCE ID NO 207
   (A) LENGTH:
   (B) TYPE: amino acid
   (D) TOPOLOGY: linear
   -E-K-S-A-S-F-R-E-E-
INFORMATION FOR SEQUENCE ID NO 208
   (A) LENGTH.
   (B) TYPE: amino acid
   (D) TOPOLOGY. linear
   -E-K-S-A-R-F-K-E-E-
INFORMATION FOR SEQUENCE ID NO 209
   (A) LENGTH:
   (B) TYPE: amino acid
   (D) TOPOLOGY: linear
   -E-E-A-R-K-K-
INFORMATION FOR SEQUENCE ID NO 210
   (A) LENGTH.
   (B) TYPE: amino acid
   (D) TOPOLOGY. linear
   -E-E-A-R-E-K-
INFORMATION FOR SEQUENCE ID NO 211
   (A) LENGTH:
   (B) TYPE: amino acid
   (D) TOPOLOGY: linear
   -K-T-Q-T-K-G-K-
INFORMATION FOR SEQUENCE ID NO 212
   (A) LENGTH:
   (B) TYPE: amino acid
   (D) TOPOLOGY: linear
   -K-T-Q-T-K-G-E-
INFORMATION FOR SEQUENCE ID NO 213
   (A) LENGTH:
   (B) TYPE: amino acid
   (D) TOPOLOGY: linear
   -E-G-S-V-D-G-D-T-R-
INFORMATION FOR SEQUENCE ID NO 214
   (A) LENGTH:
   (B) TYPE: amino acid
   (D) TOPOLOGY: linear
   -E-G-S-V-D-E-D-T-K-

### ALZHEIMER AMYLOID A4

INFORMATION FOR SEQUENCE ID NO 215
   (A) LENGTH:
   (B) TYPE: amino acid
   (D) TOPOLOGY: linear
   -D-S-A-D-A-E-E-D-D-
INFORMATION FOR SEQUENCE ID NO 216
   (A) LENGTH
   (B) TYPE: amino acid
   (D) TOPOLOGY: linear
   -E-E-E-E-V-A-E-V-E-
INFORMATION FOR SEQUENCE ID NO 217
   (A) LENGTH
   (B) TYPE: amino acid
   (D) TOPOLOGY: linear
   -E-E-E-E-A-D-D-D-
INFORMATION FOR SEQUENCE ID NO 218
   (A) LENGTH:
   (B) TYPE: amino acid
   (D) TOPOLOGY: linear
   A-D-D-D-E-D-D-E-D-G-
INFORMATION FOR SEQUENCE ID NO 219
   (A) LENGTH
   (B) TYPE: amino acid
   (D) TOPOLOGY: linear
   -D-E-D-G-D-E-V-E-
INFORMATION FOR SEQUENCE ID NO 220
   (A) LENGTH:
   (B) TYPE amino acid
   (D) TOPOLOGY: linear
   -E-E-A-E-E-P-Y-E-E-
INFORMATION FOR SEQUENCE ID NO 221
   (A) LENGTH:
   (B) TYPE: amino acid
   (D) TOPOLOGY: linear
   -A-D-V-E-E-E-E-A-D-D-D-E-D-V-E-
INFORMATION FOR SEQUENCE ID NO 222
   (A) LENGTH:
   (B) TYPE: amino acid
   (D) TOPOLOGY: linear
   -K-A-K-E-R-L-E-
INFORMATION FOR SEQUENCE ID NO 223
   (A) LENGTH:
   (B) TYPE: amino acid
   (D) TOPOLOGY: linear
   -E-A-K-H-R-E-R-
INFORMATION FOR SEQUENCE ID NO 224
   (A) LENGTH:
   (B) TYPE: amino acid
   (D) TOPOLOGY: linear
   -R-E-W-E-E-A-E-R-
INFORMATION FOR SEQUENCE ID NO 225
   (A) LENGTH:
   (B) TYPE: amino acid
   (D) TOPOLOGY: linear
   -R-A-E-Q-K-D-R-

### APOLIPOPROTEIN E

INFORMATION FOR SEQUENCE ID NO 226
   (A) LENGTH
   (B) TYPE: amino acid
   (D) TOPOLOGY. linear
   -E-P-E-T-E-P-D-V-R-
INFORMATION FOR SEQUENCE ID NO 227
   (A) LENGTH:
   (B) TYPE, amino acid
   (D) TOPOLOGY linear
   -E-G-E-P-E-V-T-D-
INFORMATION FOR SEQUENCE ID NO 228
   (A) LENGTH
   (B) TYPE amino acid
   (D) TOPOLOGY. linear
   -E-T-E-P-E-P-E-L-R-
INFORMATION FOR SEQUENCE ID NO 229
   (A) LENGTH:
   (B) TYPE: amino acid
   (D) TOPOLOGY: linear
   -E-D-V-C-G-R-
INFORMATION FOR SEQUENCE ID NO 230
   (A) LENGTH:
   (B) TYPE: amino acid
   (D) TOPOLOGY: linear
   -E-D-V-R-G-R-
INFORMATION FOR SEQUENCE ID NO 231
   (A) LENGTH:
   (B) TYPE: amino acid
   (D) TOPOLOGY: linear
   -E-D-L-R-N-R-
INFORMATION FOR SEQUENCE ID NO 232
   (A) LENGTH:
   (B) TYPE: ammo acid
   (D) TOPOLOGY: linear
   -H-L-R-K-L-R-K-R-
INFORMATION FOR SEQUENCE ID NO 233
   (A) LENGTH:
   (B) TYPE: amino acid
   (D) TOPOLOGY: linear
   -H-P-R-K-M-K-R-R-
INFORMATION FOR SEQUENCE ID NO 234
   (A) LENGTH:
   (B) TYPE: amino acid
   (D) TOPOLOGY: linear
   -H-L-R-K-M-R-K-R-
INFORMATION FOR SEQUENCE ID NO 235
   (A) LENGTH:
   (B) TYPE: amino acid
   (D) TOPOLOGY: linear
   -R-D-R-L-D-E-V-K-E-
INFORMATION FOR SEQUENCE ID NO 236
   (A) LENGTH:
   (B) TYPE: amino acid
   (D) TOPOLOGY: linear
   -R-A-R-M-E-E-
INFORMATION FOR SEQUENCE ID NO 237
   (A) LENGTH:
   (B) TYPE: amino acid
   (D) TOPOLOGY linear
   -R-G-R-M-E-E-
INFORMATION FOR SEQUENCE ID NO 238
   (A) LENGTH
   (B) TYPE: amino acid
   (D) TOPOLOGY linear
   -R-G-R-L-E-E-

### HERPES VIRUS 1 (HSV1) GLYCOPROTEIN B

INFORMATION FOR SEQUENCE ID NO 239
   (A) LENGTH:
   (B) TYPE: amino acid
   (D) TOPOLOGY: linear
   -D-Y-T-E-V-Q-R-R-N-Q-L-H-D-
INFORMATION FOR SEQUENCE ID NO 240
   (A) LENGTH.
   (B) TYPE: amino acid
   (D) TOPOLOGY linear
   -K-E-L-K-N-P-T-N-P-D-
INFORMATION FOR SEQUENCE ID NO 242
   (A) LENGTH:
   (B) TYPE: amino acid
   (D) TOPOLOGY: linear
   -E-G-E-E-G-G-D-F-D-E-A-K-
INFORMATION FOR SEQUENCE ID NO 242
   (A) LENGTH:
   (B) TYPE: amino acid
   (D) TOPOLOGY: linear
   -D-E-A-K-L-A-E-A-R-E-M-I-R-
INFORMATION FOR SEQUENCE ID NO 243
   (A) LENGTH:
   (B) TYPE: amino acid
   (D) TOPOLOGY: linear
   -D-M-V-M-R-K-R-R-N-
INFORMATION FOR SEQUENCE ID NO 244
   (A) LENGTH:
   (B) TYPE: amino acid
   (D) TOPOLOGY: linear
   -R-K-R-R-N-T-N-Y-T-Q-V-P-N-K-D-A-D-E-D-D-L-

### HERPES VIRUS 2 (HSV 23, 2H) GLYCOPROTEIN B

INFORMATION FOR SEQUENCE ID NO 245
   (A) LENGTH:
   (B) TYPE: amino acid
   (D) TOPOLOGY: linear
   -K-A-R-K-R-K-T-K-K-
INFORMATION FOR SEQUENCE ID NO 246
   (A) LENGTH
   (B) TYPE: amino acid
   (D) TOPOLOGY linear
   -K-K-P-P-K-R-P-E-
INFORMATION FOR SEQUENCE ID NO 247
   (A) LENGTH:
   (B) TYPE: amino acid
   (D) TOPOLOGY: linear
   -R-D-A-R-E-A-I-D-R-
INFORMATION FOR SEQUENCE ID NO 248
   (A) LENGTH:
   (B) TYPE: amino acid
   (D) TOPOLOGY: linear
   -R-E-Y-M-R-E-Q-D-R-K-
INFORMATION FOR SEQUENCE ID NO 249
   (A) LENGTH:
   (B) TYPE: amino acid
   (D) TOPOLOGY: linear
   -R-K-R-N-K-A-R-
INFORMATION FOR SEQUENCE ID NO 250
   (A) LENGTH
   (B) TYPE amino acid
   (D) TOPOLOGY: linear
   -E-D-E-A-G-D-E-D-E-L-
INFORMATION FOR SEQUENCE ID NO 251
   (A) LENGTH:
   (B) TYPE: amino acid
   (D) TOPOLOGY: linear
   -R-K-R-N-K-A-R-Y-S-P-L-H-N-E-D-E-A-G-D-E-D-E-L-

### TREPONEMA PALLIDUM MEMBRANE PROTEIN TMPA

INFORMATION FOR SEQUENCE ID NO 252
   (A) LENGTH
   (B) TYPE amino acid
   (D) TOPOLOGY linear
   -K-E-E-A-E-K-K-A-A-E-Q-R-
INFORMATION FOR SEQUENCE ID NO 253
   (A) LENGTH:
   (B) TYPE: amino acid
   (D) TOPOLOGY: linear
   -H-A-D-R-R-L-M-E-
INFORMATION FOR SEQUENCE ID NO 254
   (A) LENGTH:
   (B) TYPE: amino acid
   (D) TOPOLOGY: linear
   -D-V-E-R-Q-S-T-D-A-K-
INFORMATION FOR SEQUENCE ID NO 255
   (A) LENGTH:
   (B) TYPE amino acid
   (D) TOPOLOGY: linear
   -K-I-E-G-D-L-K-K-
INFORMATION FOR SEQUENCE ID NO 256
   (A) LENGTH
   (B) TYPE: amino acid
   (D) TOPOLOGY: linear
   -E-L-R-P-Q-L-E-E-A-D-
INFORMATION FOR SEQUENCE ID NO 257
   (A) LENGTH
   (B) TYPE: amino acid
   (D) TOPOLOGY linear
   -D-S-Q-Y-Q-E-A-A-E-A-E-E-
INFORMATION FOR SEQUENCE ID NO 258
   (A) LENGTH
   (B) TYPE amino acid
   (D) TOPOLOGY linear
   -R-L-K-K-T-E-A-E-K-
INFORMATION FOR SEQUENCE ID NO 259
   (A) LENGTH:
   (B) TYPE: amino acid
   (D) TOPOLOGY: linear
   -A-K-T-K-Q-K-A-
INFORMATION FOR SEQUENCE ID NO 260
   (A) LENGTH:
   (B) TYPE: amino acid
   (D) TOPOLOGY: linear
   -D-L-A-R-S-A-D-K-S-
INFORMATION FOR SEQUENCE ID NO 261
   (A) LENGTH:
   (B) TYPE: amino acid
   (D) TOPOLOGY: linear
   -K-E-P-I-E-V-E-P-L- P-N-D-R-
INFORMATION FOR SEQUENCE ID NO 262
   (A) LENGTH
   (B) TYPE: amino acid
   (D) TOPOLOGY. linear
   -R-V-Q-S-R-G-V-E-D-G-G-R-S-P-K-

### ISLET AMYLOID POLYPEPTIDE

INFORMATION FOR SEQUENCE ID NO 263
   (A) LENGTH
   (B) TYPE: amino acid
   (D) TOPOLOGY: linear
   -E-S-H-Q-V-E-K-R-K-
INFORMATION FOR SEQUENCE ID NO 264
   (A) LENGTH
   (B) TYPE - amino acid
   (D) TOPOLOGY. linear
   -N-P-Q-M-D-K-R-K-
INFORMATION FOR SEQUENCE ID NO 265
   (A) LENGTH:
   (B) TYPE: amino acid
   (D) TOPOLOGY: linear
   -D-H-R-V-D-K-R-K-
INFORMATION FOR SEQUENCE ID NO 266
   (A) LENGTH:
   (B) TYPE: amino acid
   (D) TOPOLOGY: linear
   -K-R-N-A-V-E-V-L-K-R-E-
INFORMATION FOR SEQUENCE ID NO 267
   (A) LENGTH:
   (B) TYPE: amino acid
   (D) TOPOLOGY: linear
   -K-R-N-V-A-E-D-P-N-R-E-
INFORMATION FOR SEQUENCE ID NO 268
   (A) LENGTH:
   (B) TYPE: amino acid
   (D) TOPOLOGY: linear
   -R-R-N-A-E-V-V-D-V-E-

### COLLAGENASE (FIBROBLAST MMP1)

INFORMATION FOR SEQUENCE ID NO 269
   (A) LENGTH:
   (B) TYPE: amino acid
   (D) TOPOLOGY: linear
   -L-K-N-D-G-R-Q-V-E-K-R-R-N-
INFORMATION FOR SEQUENCE ID NO 270
   (A) LENGTH:
   (B) TYPE: amino acid
   (D) TOPOLOGY linear -K-N-D-G-R-Q-V-E-K-R-R-
INFORMATION FOR SEQUENCE ID NO 271
   (A) LENGTH:
   (B) TYPE: amino acid
   (D) TOPOLOGY: linear
   -K-D-D-W-R-K-I-P-K-Q-R-
INFORMATION FOR SEQUENCE ID NO 272
   (A) LENGTH:
   (B) TYPE: amino acid
   (D) TOPOLOGY: linear
   -K-K-V-E-R-Q-R-
INFORMATION FOR SEQUENCE ID NO 273
   (A) LENGTH:
   (B) TYPE: amino acid
   (D) TOPOLOGY: linear
   -D-G-V-P-V-E-K-K-R-
INFORMATION FOR SEQUENCE ID NO 274
   (A) LENGTH.
   (B) TYPE ammo acid
   (D) TOPOLOGY: linear
   -R-G-D-H-R-D-N-
INFORMATION FOR SEQUENCE ID NO 275
   (A) LENGTH:
   (B) TYPE: amino acid
   (D) TOPOLOGY. linear
   -D-E-H-E-R-W-
INFORMATION FOR SEQUENCE ID NO 276
   (A) LENGTH:
   (B) TYPE: amino acid
   (D) TOPOLOGY linear
   -D-E-D-D-R-W-T-K-D-
INFORMATION FOR SEQUENCE ID NO 277
   (A) LENGTH:
   (B) TYPE: amino acid
   (D) TOPOLOGY: linear
   -E-F-A-D-R-D-E-V-R-
INFORMATION FOR SEQUENCE ID NO 278
   (A) LENGTH:
   (B) TYPE: amino acid
   (D) TOPOLOGY: linear
   -E-V-A-H-R-D-E-
INFORMATION FOR SEQUENCE ID NO 279
   (A) LENGTH:
   (B) TYPE: amino acid
   (D) TOPOLOGY: linear
   -E-V-A-D-R-D-E-V-R-
INFORMATION FOR SEQUENCE ID NO 280
   (A) LENGTH:
   (B) TYPE: amino acid
   (D) TOPOLOGY: linear
   -R-Y-D-E-Y-K-R-

### SCHISTOSOMA ELASTASE PRECURSOR

INFORMATION FOR SEQUENCE ID NO 281
   (A) LENGTH:
   (B) TYPE: amino acid
   (D) TOPOLOGY: linear
   -R-S-G-E-P-
INFORMATION FOR SEQUENCE ID NO 282
   (A) LENGTH
   (B) TYPE: amino acid
   (D) TOPOLOGY linear
   -R-N-G-D-Q-Q-G-I-H-H-
INFORMATION FOR SEQUENCE ID NO 283
   (A) LENGTH:
   (B) TYPE: amino acid
   (D) TOPOLOGY: linear
   -A-R-Q-R-R-P-
INFORMATION FOR SEQUENCE ID NO 284
   (A) LENGTH:
   (B) TYPE: amino acid
   (D) TOPOLOGY: linear
   -R-D-D-N-D-R-D-P-S-A-K-

### SCHISTOSOMIN

INFORMATION FOR SEQUENCE ID NO 285
   (A) LENGTH
   (B) TYPE: amino acid
   (D) TOPOLOGY: linear
   -K-V-S-K-R-P-D-Y-D-
INFORMATION FOR SEQUENCE ID NO 286
   (A) LENGTH:
   (B) TYPE: amino acid
   (D) TOPOLOGY linear
   -E-A-F-E-C-F-E-S-D-P-N-A-K-
INFORMATION FOR SEQUENCE ID NO 287
   (A) LENGTH:
   (B) TYPE: amino acid
   (D) TOPOLOGY: linear
   -K-C-R-K-K-Y-E-F-C-R-

### APOLIPOPROTEIN(a) HUMAN

INFORMATION FOR SEQUENCE ID NO 288
   (A) LENGTH
   (B) TYPE: amino acid
   (D) TOPOLOGY linear
   -H-R-H-Q-R-T-P-E-N-Y-P-N-D-
INFORMATION FOR SEQUENCE ID NO 289
   (A) LENGTH:
   (B) TYPE: amino acid
   (D) TOPOLOGY: linear
   -D-P-S-I-R-W-E-Y-C-

### APOLIPOPROTEIN(a) RHESUS

INFORMATION FOR SEQUENCE ID NO 290
   (A) LENGTH:
   (B) TYPE: amino acid
   (D) TOPOLOGY: linear
   -H-Q-H-K-R-T-P-E-N-H-P-N-D-D-
INFORMATION FOR SEQUENCE ID NO 291
   (A) LENGTH:
   (B) TYPE: amino acid
   (D) TOPOLOGY: linear
   -M-D-P-S-V-R-R-E-Y-C-
INFORMATION FOR SEQUENCE ID NO 292
   (A) LENGTH:
   (B) TYPE: amino acid
   (D) TOPOLOGY linear
   -R-T-P-E-N-Y-P-N-G-
INFORMATION FOR SEQUENCE ID NO 293
   (A) LENGTH:
   (B) TYPE: amino acid
   (D) TOPOLOGY linear
   -L-E-A-P-S-K-Q-A-
INFORMATION FOR SEQUENCE ID NO 294
   (A) LENGTH
   (B) TYPE amino acid
   (D) TOPOLOGY linear
   -L-E-A-F-L-Q-E-P-T-E-E-
INFORMATION FOR SEQUENCE ID NO 295
   (A) LENGTH:
   (B) TYPE: amino acid
   (D) TOPOLOGY: linear
   -G-H-L-A-G-G-T-D-R-

### HEPATITIS DELTA ANTIGEN

INFORMATION FOR SEQUENCE ID NO 296
   (A) LENGTH:
   (B) TYPE: amino acid
   (D) TOPOLOGY: linear
   -E-R-R-K-D-R-G-G-R-E-D-
INFORMATION FOR SEQUENCE ID NO 297
   (A) LENGTH:
   (B) TYPE: amino acid
   (D) TOPOLOGY: linear
   -E-S-R-K-N-R-G-G-R-E-E-
INFORMATION FOR SEQUENCE ID NO 298
   (A) LENGTH.
   (B) TYPE. amino acid
   (D) TOPOLOGY. linear
   -E-S-K-G-K-R-A-G-R-E-Q-

### REV PROTEIN HIV, SIV, VILV, OMVVS

INFORMATION FOR SEQUENCE ID NO 299
   (A) LENGTH.
   (B) TYPE: amino acid
   (D) TOPOLOGY linear
   -K-E-G-F-R-N-K-V-P-C-L-Q-E-
INFORMATION FOR SEQUENCE ID NO 300
   (A) LENGTH.
   (B) TYPE amino acid
   (D) TOPOLOGY linear
   -G-E-E-E-L-R-R-R-L-R-
INFORMATION FOR SEQUENCE ID NO 301
   (A) LENGTH:
   (B) TYPE: amino acid
   (D) TOPOLOGY: linear
   -H-E-R-E-E-E-L-R-K-R-L-R-
INFORMATION FOR SEQUENCE ID NO 302
   (A) LENGTH:
   (B) TYPE: amino acid
   (D) TOPOLOGY: linear
   -T-E-E-E-L-R-R-R-L R-
INFORMATION FOR SEQUENCE ID NO 303
   (A) LENGTH:
   (B) TYPE: amino acid
   (D) TOPOLOGY: linear
   -N-E-E-E-L-R-R-R-L-R-
INFORMATION FOR SEQUENCE ID NO 304
   (A) LENGTH
   (B) TYPE amino acid
   (D) TOPOLOGY: linear
   -E-R-A-D-E-E-G-L-O-G-K-L-R-
INFORMATION FOR SEQUENCE ID NO 305
   (A) LENGTH:
   (B) TYPE: amino acid
   (D) TOPOLOGY: linear
   -D-S-D-E-E-L-L-R-T-V-R-
INFORMATION FOR SEQUENCE ID NO 306
   (A) LENGTH:
   (B) TYPE: amino acid
   (D) TOPOLOGY: linear
   -E-P-Q-D-D-A-R-L-L-Q-A-V-K-
INFORMATION FOR SEQUENCE ID NO 307
   (A) LENGTH:
   (B) TYPE: amino acid
   (D) TOPOLOGY: linear
   -P-E-E-R-R-L-L-B-
INFORMATION FOR SEQUENCE ID NO 308
   (A) LENGTH:
   (B) TYPE: amino acid
   (D) TOPOLOGY: linear
   -W-R-D-M-E-P-P-L-R-E-
INFORMATION FOR SEQUENCE ID NO 309
   (A) LENGTH:
   (B) TYPE: amino add
   (D) TOPOLOGY: linear
   -W-R-D-M-E-P-P-L-R-E-
INFORMATION FOR SEQUENCE ID NO 310
   (A) LENGTH.
   (B) TYPE. amino acid
   (D) TOPOLOGY linear
   -Q-K-S-M-E-P-P-L-R-E-
INFORMATION FOR SEQUENCE ID NO 311
   (A) LENGTH
   (B) TYPE: amino acid
   (D) TOPOLOGY: linear
   -K-K-K-R-G-W-Y-K-W-L-R-K-L-
INFORMATION FOR SEQUENCE ID NO 312
   (A) LENGTH:
   (B) TYPE. amino acid
   (D) TOPOLOGY linear
   -K-R-R-K-G-W-F-Q-W-L-R-K-L-

### ANGIOTENSINOGEN

INFORMATION FOR SEQUENCE ID NO 313
   (A) LENGTH:
   (B) TYPE: amino acid
   (D) TOPOLOGY: linear
   -D-R-V-Y-I-H-P-F-H-L-L-Y-H-N -K-
INFORMATION FOR SEQUENCE ID NO 314
   (A) LENGTH:
   (B) TYPE: amino acid
   (D) TOPOLOGY: linear
   -D-R-V-Y-I-H-P-F-H-L-L-Y-Y-S-K-
INFORMATION FOR SEQUENCE ID NO 315
   (A) LENGTH:
   (B) TYPE: amino acid
   (D) TOPOLOGY linear
   -D-R-V-Y-I-H-P-F-H-L V-I-H-N-E-
INFORMATION FOR SEQUENCE ID NO 316
   (A) LENGTH
   (B) TYPE amino acid
   (D) TOPOLOGY linear
   -E-N-P-S-V-E-T-L-P-E-S-T-F-
INFORMATION FOR SEQUENCE ID NO 317
   (A) LENGTH.
   (B) TYPE: amino acid
   (D) TOPOLOGY linear
   -E-N-P-S-V-E-T-L-P-E-P-T-F-
INFORMATION FOR SEQUENCE ID NO 318
   (A) LENGTH
   (B) TYPE: amino acid
   (D) TOPOLOGY linear
   -A-K-A-N-A-G-K-P-K-D-P-T-F-
INFORMATION FOR SEQUENCE ID NO 319
   (A) LENGTH:
   (B) TYPE: amino acid
   (D) TOPOLOGY: linear
   -K-I-S-N-D-R-I-R-V-G-E-
INFORMATION FOR SEQUENCE ID NO 320
   (A) LENGTH:
   (B) TYPE: amino acid
   (D) TOPOLOGY: linear
   -K-M-G-D-T-N-P-R-V-G-E-
INFORMATION FOR SEQUENCE ID NO 321
   (A) LENGTH
   (B) TYPE: amino acid
   (D) TOPOLOGY: linear
   -N-I-G-D-T-N-P-R-V-G-E-
INFORMATION FOR SEQUENCE ID NO 322
   (A) LENGTH:
   (B) TYPE: amino acid
   (D) TOPOLOGY linear
   -E-L-E-A-D-E-R-E-P-T-E-

### PRORENIN

INFORMATION FOR SEQUENCE ID NO 323
   (A) LENGTH.
   (B) TYPE: amino acid
   (D) TOPOLOGY linear
   -D-G-W-R-R-M-P-
INFORMATION FOR SEQUENCE ID NO 324
   (A) LENGTH:
   (B) TYPE: amino acid
   (D) TOPOLOGY linear
   -D-R-R-R-M-P-
INFORMATION FOR SEQUENCE ID NO 325
   (A) LENGTH:
   (B) TYPE: amino acid
   (D) TOPOLOGY: linear
   -K-R-I-F-L-K-R-M-P-S-I-R-E-
INFORMATION FOR SEQUENCE ID NO 326
   (A) LENGTH:
   (B) TYPE: amino acid
   (D) TOPOLOGY: linear
   -G-R-I-L-L-K-K-M-P-S-V-R-E-
INFORMATION FOR SEQUENCE ID NO 327
   (A) LENGTH:
   (B) TYPE amino acid
   (D) TOPOLOGY: linear
   -K-R-M-P-S-I-R-E-S-L-K-E-R-G-
INFORMATION FOR SEQUENCE ID NO 328
   (A) LENGTH
   (B) TYPE. amino acid
   (D) TOPOLOGY: linear
   -K-K-M-P-S-V-R-E-I-L-E-E-R-G-
INFORMATION FOR SEQUENCE ID NO 329
   (A) LENGTH.
   (B) TYPE: amino acid
   (D) TOPOLOGY linear
   -R-E-I-L-E-E-R-G-
INFORMATION FOR SEQUENCE ID NO 330
   (A) LENGTH.
   (B) TYPE amino acid
   (D) TOPOLOGY. linear
   - R-L-G-P-E-W-S-Q-P-M-K-R-
INFORMATION FOR SEQUENCE ID NO 331
   (A) LENGTH:
   (B) TYPE: amino acid
   (D) TOPOLOGY: linear
   -R-I-S-A -E-W-G-E-F-I-K-K-
INFORMATION FOR SEQUENCE ID NO 332
   (A) LENGTH:
   (B) TYPE: amino acid
   (D) TOPOLOGY: linear
   -R-I-S-A-E-W-G-E-F-I-K-K-
INFORMATION FOR SEQUENCE ID NO 333
   (A) LENGTH:
   (B) TYPE: amino acid
   (D) TOPOLOGY: linear
   -R-L-S-A- E-W-G- V-F-T-K-R-
INFORMATION FOR SEQUENCE ID NO 334
   (A) LENGTH
   (B) TYPE amino acid
   (D) TOPOLOGY: linear
   -H-K-L-F-D-A-S-D-S-S-S-Y-K-H-
INFORMATION FOR SEQUENCE ID NO 335
   (A) LENGTH:
   (B) TYPE: amino acid
   (D) TOPOLOGY linear
   -H-N-L-Y-D-S-S-E-S-S-S-Y-M-E-
INFORMATION FOR SEQUENCE ID NO 336
   (A) LENGTH
   (B) TYPE amino acid
   (D) TOPOLOGY linear
   -H-S-L-Y-E-S-S-D-S-S-S-Y-M-E-
INFORMATION FOR SEQUENCE ID NO 337
   (A) LENGTH:
   (B) TYPE: amino acid
   (D) TOPOLOGY: linear
   -K-E-D-V-F-S-F-S-F-Y-Y-N-R-D-S-E-N-
INFORMATION FOR SEQUENCE ID NO 338
   (A) LENGTH:
   (B) TYPE amino acid
   (D) TOPOLOGY: linear
   -K-E-E-V-F-S-V-Y-Y-N-R-G-S-H-
INFORMATION FOR SEQUENCE ID NO 339
   (A) LENGTH:
   (B) TYPE amino acid
   (D) TOPOLOGY: linear
   -D-P-Q-H-Y-E-G-N-F-H-
INFORMATION FOR SEQUENCE ID NO 340
   (A) LENGTH
   (B) TYPE: amino acid
   (D) TOPOLOGY: linear
   -E-K-L-M-E-A-L-G-A-K-K-R-L-F-D-
INFORMATION FOR SEQUENCE ID NO 341
   (A) LENGTH
   (B) TYPE amino acid
   (D) TOPOLOGY: linear
   -Q-L-I-M-Q-A-L-G-V-K-E-K-R-A-N-
INFORMATION FOR SEQUENCE ID NO 342
   (A) LENGTH:
   (B) TYPE amino acid
   (D) TOPOLOGY. linear
   -K-L-I-M-Q-A-L-G-A-K-E-K-R-I-E-
INFORMATION FOR SEQUENCE ID NO 343
   (A) LENGTH:
   (B) TYPE: amino acid
   (D) TOPOLOGY: linear
   -D-Y-V-F-Q-E-S-Y-S-S-K-K-
INFORMATION FOR SEQUENCE ID NO 344
   (A) LENGTH
   (B) TYPE: amino acid
   (D) TOPOLOGY: linear
   -D-Y-V-Q-K-N-P-F-R-N-D-D-
INFORMATION FOR SEQUENCE ID NO 345
   (A) LENGTH.
   (B) TYPE: amino acid
   (D) TOPOLOGY linear
   -D-Y-V-Q-Y-P-N-R-R-D-K-
INFORMATION FOR SEQUENCE ID NO 346
   (A) LENGTH
   (B) TYPE amino acid
   (D) TOPOLOGY linear
   -R-K-F-Y-T-E-F-D-R-R-N-N-R-
INFORMATION FOR SEQUENCE ID NO 347
   (A) LENGTH:
   (B) TYPE: amino acid
   (D) TOPOLOGY linear
   -R-K-F-Y-T-E-F-D-R-H-N-N-R-
INFORMATION FOR SEQUENCE ID NO 348
   (A) LENGTH
   (B) TYPE: amino acid
   (D) TOPOLOGY linear
   -R-K-F-Y-T-E-F-D-R-H-N-N-R-

### CORTICOTROPIN RELEASING FACTOR BINDING PROTEIN

INFORMATION FOR SEQUENCE ID NO 349
   (A) LENGTH:
   (B) TYPE: amino acid
   (D) TOPOLOGY: linear
   -R-Y-L-E-L-R-E-A-A-D-Y-D-
INFORMATION FOR SEQUENCE ID NO 350
   (A) LENGTH:
   (B) TYPE: amino acid
   (D) TOPOLOGY: linear
   -R-Y-L-E-V-Q-E-A-A-V-Y-D-
INFORMATION FOR SEQUENCE ID NO 351
   (A) LENGTH:
   (B) TYPE: amino acid
   (D) TOPOLOGY: linear
   -K-R-D-V-A-G-E-Q-P-Y-R-R-
INFORMATION FOR SEQUENCE ID NO 352
   (A) LENGTH.
   (B) TYPE: amino acid
   (D) TOPOLOGY linear
   -K-R-N-L-A-E-E-Q-P-Y-R-R-
INFORMATION FOR SEQUENCE ID NO 353
   (A) LENGTH:
   (B) TYPE: amino acid
   (D) TOPOLOGY: linear
   -D-H-P-L-P-S-A-E-R-
INFORMATION FOR SEQUENCE ID NO 354
   (A) LENGTH
   (B) TYPE: amino acid
   (D) TOPOLOGY: linear
   -D-H-P-L-P-T-R-E-R-
INFORMATION FOR SEQUENCE ID NO 355
   (A) LENGTH:
   (B) TYPE: amino acid
   (D) TOPOLOGY: linear
   -R-R-S-I-R-S-S-Q-
INFORMATION FOR SEQUENCE ID NO 356
   (A) LENGTH:
   (B) TYPE: amino add
   (D) TOPOLOGY: linear
   -R-R-S-V-T-S-S-Q-
INFORMATION FOR SEQUENCE ID NO 367
   (A) LENGTH:
   (B) TYPE: amino acid
   (D) TOPOLOGY: linear
   -D-P-S-K-M-T-P-L-A-D-
INFORMATION FOR SEQUENCE ID NO 358
   (A) LENGTH:
   (B) TYPE: amino acid
   (D) TOPOLOGY: linear
   -D-T-S-K-M-M-L-L-V-D-
INFORMATION FOR SEQUENCE ID NO 359
   (A) LENGTH:
   (B) TYPE: amino acid
   (D) TOPOLOGY: linear
   -E-P-Y-E-L-E-N-P-N-G-
INFORMATION FOR SEQUENCE ID NO 360
   (A) LENGTH:
   (B) TYPE: amino acid
   (D) TOPOLOGY linear
   -E-P-L-E-L-E-T-S-T-R-

## Claims

1. An oligopeptide consisting of an amino acid sequence Ser-Gln-Asp-Glu-Val-Arg-Glu-Asn as shown in SEQ ID No. 42 or Glu-Leu-Ser-Arg-Glu-Ser-Arg-Glu-Gly-Arg as shown in SEQ ID No. 44.

2. A pharmaceutical composition comprising an oligopeptide of claim 1.

3. The oligopeptide of claim 1 for use in therapy.

## Patentansprüche

1. Ein Oligopeptid, das aus der in der SEQ ID No. 42 gezeigten Aminosäuresequenz Ser-Gln-Asp-Glu-Val-Arg-Glu-Asn oder der in der SEQ ID No. 44 gezeigten Aminosäuresequenz Glu-Leu-Ser-Arg-Glu-Ser-Arg-Glu-Gly-Arg besteht.

2. Eine pharmazeutische Zusammensetzung, die das Oligopeptid nach Anspruch 1 umfasst.

3. Das Oligopeptid nach Anspruch 1 für die Verwendung in der Therapie.

## Revendications

1. Oligopeptide consistant en une séquence d'acides aminés Ser-Gln-Asp-Glu-Val-Arg-Glu-Asn telle que représentée dans SEQ ID n° 42 ou Glu-Leu-Ser-Arg-Glu-Ser-Arg-Glu-Gly-Arg telle que représentée dans SEQ ID n° 44.

2. Composition pharmaceutique comprenant un oligopeptide selon la revendication 1.

3. Oligopeptide selon la revendication 1 destiné à être utilisé en thérapie.
